# EUROPÄISCHE PATENTSCHRIFT

(11) **EP 1 587 824 B1**
(45) Veröffentlichungstag und Bekanntmachung des Hinweises auf die Patenterteilung: **31.12.2014**
(21) Anmeldenummer: 04704587.7
(22) Anmeldetag: 23.01.2004
(51) Int. Cl.: C07K 1/18, C12N 9/96, C12N 9/00

(54) **VERFAHREN ZUR VEREDELUNG KONZENTRIETER ENZYMLÖSUNGEN**
METHODS FOR REFINING CONCENTRATED ENZYME SOLUTIONS
PROCEDES POUR AMELIORER DES SOLUTIONS ENZYMATIQUES CONCENTREES

(30) Priorität: 31.01.2003 DE 10304066
(43) Veröffentlichungstag der Anmeldung: 26.10.2005
(73) Patentinhaber: BASF SE, 67056 Ludwigshafen (DE)
(72) Erfinder: BAUR, Dieter, E-08340 Vilassar de Mar (ES); PICHLER, Werner, A-6250 Kundl (AT); RÄHSE, Wilfried, 40589 Düsseldorf (DE); VAN HOLT, Jens, 46509 Xanten (DE)
(86) Internationale Anmeldenummer: PCT/EP2004/000551
(87) Internationale Veröffentlichungsnummer: WO 2004/067557

(56) Entgegenhaltungen:
- EP-A- 0 322 082
- EP-A- 0 365 858
- WO-A-01/37628
- WO-A-92/04367
- WO-A-93/20208
- WO-A-95/19714
- US-A- 4 806 346
- US-A- 5 565 348

## Beschreibung

Die vorliegende Erfindung betrifft Verfahren zur Veredelung von konzentrierten technischen Enzymlösungen, die entsprechenden Verfahrensprodukte und Mittel, die auf solchen Lösungen beruhen, insbesondere Wasch- und Reinigungsmittel.

Enzyme, insbesondere solche für technische Einsatzgebiete werden heutzutage meist durch Fermentation von Mikroorganismen produziert und anschließend aus den betreffenden Medien aufgereinigt. Die über meist mehrere sequentielle Verfahrensschritte erhaltenen konzentrierten Enzymlösungen werden vielfach auch als "Flüssigenzym" bezeichnet. Flüssigenzym kann als gereinigter Rohstoff betrachtet werden, welcher entweder in flüssiger Form eingesetzt oder - seltener - in eine trockene Form überführt und dann entsprechenden Anwendungen zugeführt wird.

Wichtige technische Einsatzgebiete für Enzyme, insbesondere in flüssiger Form sind Wasch- und Reinigungsmittel, die zunehmend auch in flüssiger oder Gelform angeboten werden. Weitere Einsatzgebiete liegen beispielsweise in der der Kosmetik, wobei die Enzyme wie in Wasch- und Reinigungsmitteln als wirksame Agentien eingesetzt werden, oder der Textilherstellung und -Verarbeitung oder der Lebensmittelherstellung, wobei in erster Linie die Rohstoffe durch Einsatz der Enzyme zum eigentlichen Produkt umgesetzt werden.

Reinigungs- oder Anreicherungsverfahren zur Gewinnung konzentrierter Enzymlösungen sind im Stand der Technik ausführlich beschrieben. Wichtige Ziele sind hierbei die Abtrennung der Biomasse, das heißt der insbesondere makromolekularen Bestandteile der Wirtsorganisrnen, die Abtrennung niedermolekularer Begleitstoffe und Verunreinigungen, insbesondere Medienbestandteile und Metaboliten, und die Abtrennung anderer Proteine, insbesondere Enzyme. Gleichzeitig soll das interessierende Produkt in möglichst großer Menge, Reinheit und einer möglichst hohen Aktivität erhalten werden. Andererseits enthalten die Kulturüberstände meist Faktoren, oft Peptide oder Proteine, deren Identät vielfach noch nicht bekannt ist, die aber für eine Stabilisierung des interessierenden Enzyms sorgen. Besonders vorteilhaft ist es deshalb, eine nicht völlig reine Enzymlösung zu erhalten sondern eine, die einen gewissen Anteil solcher stabilisierender Begleitstoffe enthält.

Zum Zweck der Reinigung kommen zumeist auf Filtration, Sedimentation oder Ausfällung beruhende Techniken zur Anwendung.

So sind beispielsweise zur Abtrennung der Biomasse, in der Regel nacheinander anzuwendende Verfahren entwickelt worden und im Stand der Technik etabliert. Hierzu gehören beispielsweise Separation, Mikrofiltration und Ultrafiltration. Erst danach kann im Sinne der Erfindung eigentlich von einem Enzymkonzentrat gesprochen werden.

So geht beispielsweise aus der Anmeldung WO 01/37628 A2 ein Verfahren zur Gewinnung von biotechnologisch hergestellten Wertstoffen aus Kultur- und/oder Fermenterlösungen hervor, welches das Trennen der wasserunlöslichen Feststoffe von der wäßerigen, die Wertstoffe enthaltenden Lösung, anschließendes Filtrieren der erhaltenen Lösung und Aufkonzentrieren der wertstoffhaltigen Lösung mittels Ultrafiltration umfaßt. Es ist dadurch gekennzeichnet, daß die abgetrennten Feststoffe einem Waschschritt unterzogen werden, wobei als Waschflüssigkeit das Filtrat aus der Aufkonzentrierungsstufe verwendet wird.

Weiterhin ungelöst ist jedoch das Problem, daß von der Biomasse abgetrennte Enzymkonzentrate insbesondere folgende Begleitstoffe enthalten:
1. Feststoffe, insbesondere Ausfällungen von irreversibel denaturierten Proteinen, auch von dem interessierenden Protein,
2. farbige, meist braune Verbindungen, die bei der der Fermentation vorangegangenen Sterilisation der Medienbestandteile, besonders der Stickstoff-Quellen (Maillard-Verbindungen) entstanden sind, und
3. Faktoren, die die Stabilität des interessierenden Proteins erhöhen.

Herkömmliche Veredelungsmethoden sind nur in unzureichendem Maße geeignet, aus einem von der Biomasse abgetrennten Enzymkonzentrat denaturierte Proteine und farbige Verbindungen zu entfernen; oder sie entfernen zusammen mit diesen auch einen Großteil der stabilisierenden Faktoren. Die Folge davon ist in jedem Fall eine nicht zufriedenstellende Produktqualität: Entweder weist das Enzymkonzentrat eine dunkle Farbe, Schlieren und/oder Schwebstoffe bis hin zu ausgefällten Stoffen auf oder es verfügt bei heller Farbe und klarer Lösung über eine unzureichende Enzym-Stabilität; letzteres kann meist nur zu einem gewissen Anteil durch Zugabe (teurer) stabilisierender Verbindungen ausgeglichen werden. Diese Nachteile wirken sich insbesondere auf die Produkte aus, in welche das betreffende Konzentrat eingearbeitet wird.

Beispielsweise flüssige Wasch- oder Reinigungsmittel sollten über die Zeit der Lagerung hinweg einen möglichst hohen Anteil an aktivem, das heißt stabilem Enzym enthalten. Dabei verhalten sich jedoch Wassergehalt und Stabilität umgekehrt proportional zueinander. Gleichzeitig sollten diese Mittel eine für den Anwender ansprechende Farbe und ein klares Erscheinungsbild besitzen.

Im Stand der Technik sind Verfahren zur Entfärbung von konzentrierten Enzymlösungen beschrieben. Hierzu gehören Fällungsmethoden, zum Beispiel mit organischen Lösungsmitteln oder Polymeren, insbesondere aber das Aussalzen des interessierenden Proteins mit Natriumsulfat (beschrieben in H.Ruttloff (1994): "Industrielle Enzyme" Behr's Verlag, Hamburg, Kapitel 6.3.3.6, Seiten 376 bis 379). So gehen beispielsweise aus dem Patent US 5405767 bestimmte Verbindungen hervor, die der zu fällenden Proteinlösung zugegeben werden sollen, um vorteilhafte Präzipitate zu erhalten. Hierbei wird das Protein gefällt, wobei die Begleitstoffe im Überstand bleiben. Allerdings wird durch das Ausfällen und Resuspendieren ein Teil des Proteins irreversibel denaturiert und insgesamt die Stabilität beeinträchtigt; nicht zuletzt auch deshalb, weil auch stabilisierende Verbindungen abgetrennt werden. Als zu erzielende Ausbeute werden in der Tabelle auf Seite 378 des genannten Lehrbuchs ca. 50% angegeben.

Eine weitere Alternative besteht in der adsorptiven Reinigung der Enzyme, beispielsweise über ein Ionenaustausch-Harz (H.Ruttloff (1994): "Industrielle Enzyme" Behr's Verlag, Hamburg, Kapitel 6.3.3.7 and 6.3.3.8, Seiten 379 bis 396). Hierbei binden die interessierenden Proteine an ein Chromatographie-Material und werden anschließend mit einem anderen Medium eluiert. Allerdings werden hierdurch aufgrund von Denaturierungs- und Faltungseffekten ebenfalls zumeist nur schlechte Ausbeuten erzielt. So werden für verschiedene Chromatographieverfahren (außer der Affinitätschromatographie) in der Tabelle auf Seite 378 Ausbeutewerte von maximal ca. 60% angegeben. Die spezifischen, insbesondere die Affinitätschromatographie-Materialien sind im allgemeinen leistungsfähiger, aber sehr empfindlich und aufwendig herzustellen. Letztere finden daher überwiegend in der Analytik und der Medizintechnik, in der großtechnischen Enzymherstellung jedoch praktisch keine Anwendung.

So beschreibt beispielsweise die Patentanmeldung WO 89/05863 A1 die Gewinnung extrazellulärer Enzyme aus der Fermentationsbrühe von Bacillus-Stämmen. Hierin werden Experimente zur Entfernung von Zellwand-Polymeren über eine lonenaustausch-Chromatographie beschrieben, insbesondere im Zuge einer Amylase-Präparation. Dabei wird zunächst die enzym- und polymerhaltige Lösung auf die Säule aufgebracht, dann zunächst mit Puffer gespült und anschließend mit einem Elutionsmedium eluiert. Das heißt, die zu reinigende Alpha-Amylase bindet zunächst ebenso wie die Begleitstoffe an das Chromatographie-Material und wird erst anschließend bei Erhöhung der Ionenstärke heruntergewaschen.

Der umgekehrte Ansatz, gezielt die Verunreinigungen über ein Trägermaterial aus der flüssigen Lösung abzureichern, ist bislang nur für Nahrungsmittelrohstoffe gewählt worden. So offenbart das Patent US 5972121 die Entfernung von Farbstoffen aus Zucker-Lösungen über eine schwach saure oder eine schwach basische AdsorptionsChromatographie. Auch in dem Handbuch "DIAION^{®}. Manual of ion exchange resins and synthetic adsorbent, Band II" von der Firma Mitsubishi Kasei Corp. (Tokyo, Japan), 2. Druck, 1.5.1993, wird auf den Seiten 93 bis 100 die Entfärbung von Zucker unter anderem über nacheinandergeschaltete, verschiedene lonenaustausch-Chromatographie-Schritte beschrieben. Durch diesen umgekehrten Ansatz ergeben sich grundsätzlich mehrere, im einzelnen jeweils sehr selektive Aufreinigungsschritte, bei denen die jeweils abtrennbare Verunreinigung auf dem entsprechenden Material verbleibt.

In dem Patent US 5565348, welches sich mit der Gewinnung einer bestimmten alkalischen Protease eines Bacillus befaßt, wird in einem Beispiel die Aufreinigung dieses Enzyms über einen aus zahlreichen Schritten bestehenden Reinigungsvorgang beschrieben. Hierzu gehören insbesondere die Schritte des Fällens des Proteins durch Aussalzen, der Aufnahme in einem anderen Medium und einer Dialyse, bevor eine lonenaustausch-Chromatographie durchgeführt wird; auf diese folgt wiederum eine Affinitätschromatographie, das heißt ein Chromatographieschritt, bei dem die Protease spezifisch an das Säulenmaterial bindet. Bezüglich des lonenaustausch-Chromatographie-Schritts heißt es, die beschriebene spezielle Protease binde nicht an das verwendete spezielle Chromatographie-Material und werde deshalb mit dem Durchbruch erhalten. Jedoch erscheint dies nicht wie eine verallgemeinerungsfähige Lehre zur Reinigung des Enzyms, weil über das betreffende Säulenmaterial keinerlei Angaben gemacht werden, die Konzentration des Enzyms nicht angegeben ist und die eigentliche Reinigung von Begleitstoffen bereits in der vorangegangenen Präzipitation erfolgt ist, beziehungsweise durch die nachfolgende Affinitätschromatographie gewährleistet wird. Ein Enzym, insbesondere eine Protease über ein Säulenmaterial zu reinigen, an welches sie selbst gar nicht bindet, war somit bislang im Stand der Technik noch nicht in Erwägung gezogen worden, vor allem nicht unter Verzicht auf einen Präzipitations-, das heißt Ausfällungsschritt.

Es stellte sich somit die Aufgabe, Enzymkonzentrate von Feststoffen, insbesondere von irreversibel denaturierten Proteinen zu befreien und so gezielt zu entfärben, daß gleichzeitig eine möglichst hohe Lagerstabilität der Konzentrate erhalten bleibt.

Diese Aufgabe wird durch Verfahren zur Veredelung konzentrierter Enzymlösungen gemäß Anspruch 1 gelöst.

Hierbei erfolgt keine Präzipitation. Vielmehr bleiben die interessierenden Enzymproteine das ganze Verfahren über in Lösung, wofür, wie unten und in den Beispielen ausgeführt wird, bestimmte Konzentrationsbereiche hinsichtlich der zu erzielenden Ausbeute besonders vorteilhaft sind.

Verfahrensschritt (a) gehen an sich bekannte, im Stand der Technik beschriebene Verfahren zur Herstellung weitgehend Biomasse-freier, angereicherter, wäßriger Enzymlösungen voraus. In der Regel handelt es sich dabei um mehrere Teilschritte, wie Zellaufschluß, Pelletieren der Zelltrümmer, Dekantieren und gegebenenfalls weitere Zentrifugationsschritte. Auch Separation, Mikro-, Ultra- oder Steril-Filtrationen (siehe unten) und die Einkonzentrierung, das heißt Entfernung des Lösungsmittels bis zu einem mittleren Konzentrationsbereich des Enzyms können bereits zum Einsatz kommen. Als optimal kann dabei ein Enzym-Konzentrationswert angesehen werden, der in dem Größenbereich der Hälfte des im weiteren Verfahren als optimale Arbeitskonzentration bezeichneten Bereichs liegt (siehe unten). Vorteilhaft ist ferner ein zu erzielender Schweb- oder Feststoffanteil von unter 1 Vol.-%, wie dies beispielsweise über Zentrifugation mit einer eine Tischzentrifuge für 10 min bei 7.000 g überprüft werden kann. Die betreffende Lösung sollte zudem auf einen pH-Wert eingestellt werden, der für das Enzym verträglich ist und bei dem es eine positive Ladung aufweist.

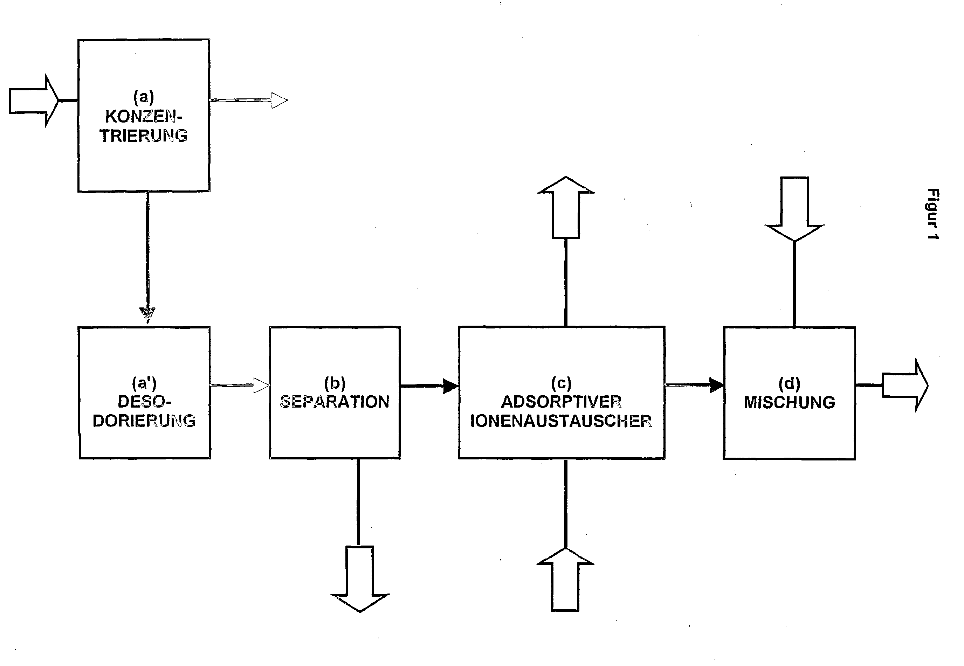

Auch Schritt (a), der in Figur 1 als "Konzentrierung" bezeichnet ist, beruht auf Methoden, die dem Fachmann an sich bekannt sind. Für die Einkonzentrierung können beispielsweise ein Rotavapor oder ein Dünnschichtverdampfer eingesetzt werden. Besonders vorteilhaft ist es dabei, wenn der pH-Wert so wie zuvor eingestellt weitgehend konstant bleibt und daß der Feststoffanteil des Enzymkonzentrats möglichst gering bleibt (siehe unten). Ansonsten steigen die Verluste im nachfolgenden Schritt (b) unvorteilhaft stark an.

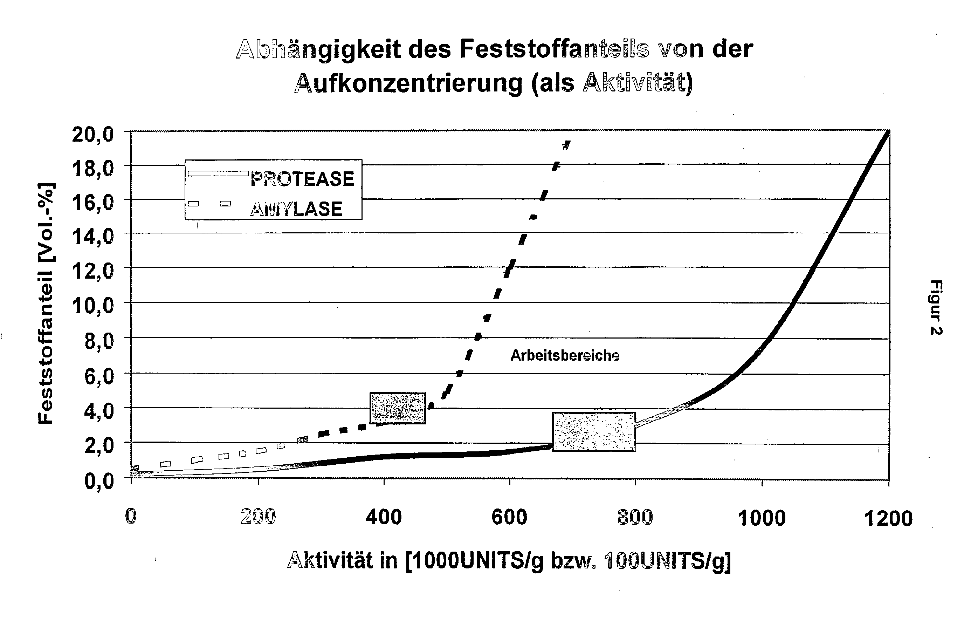

Schritt (a) soll über die bekannten Methoden (insbesondere durch rechtzeitigen Abbruch der Konzentrierung) so gesteuert werden, daß das erhaltene Enzymkonzentrat gerade noch keine (quantitative) Proteinausfällung aufweist. Der optimale Arbeitsbereich muß dabei für jedes Enzym individuell ermittelt werden, und zwar nicht nur hinsichtlich der Temperatur, dem pH-Wert und der Ionenstärke sondern insbesondere hinsichtlich eines optimalen Enzym-Konzentrationsbereichs. Dies ist in den Beispielen 1 und 2 der vorliegenden Anmeldung für die Alkalische Protease aus *Bacillus lentus* und für die α-Amylase aus *Bacillus sp.* A 7-7 (DSM 12368) vorgenommen worden. Für die untersuchte Protease wurde demnach ein optimaler Konzentrationsbereich von 700.000 bis 800.000 HPE/g und für die α-Amylase einer von 35.000 bis 45.000 TAU/g ermittelt. Oberhalb dieser Werte steigt der Anteil an ausgefällten Feststoffen in Abhängigkeit von der Aktivität bald überproportional an, was mit einem drastisch steigenden Verlust an Gutprodukt einhergeht. Diese Effekte werden für die genannten Beispielenzyme durch Figur 2 illustriert. Eine derartige Abhängigkeit des Feststoffanteils von der Konzentration an Protein, insbesondere an Enzymaktivität, ist für praktisch alle technischen Enzyme zu erwarten. Sie muß im Einzelfall experimentell ermittelt und das Verfahren über die an sich bekannten Konzentrations- und gegebenenfalls Verdünnungsverfahren darauf ausgerichtet werden.

Wie in den Beispielen ebenfalls gezeigt ist, ist es bevorzugt, diesen Arbeitsbereich das ganze Verfahren über möglichst einzuhalten, um einerseits hochkonzentriert und damit effizient zu arbeiten, andererseits aber um möglichst wenig Enzym durch Denaturierung und Ausfällung zu verlieren. Auf diese Weise konnten Ausbeuten von bis zu 95% für das gesamte Verfahren erzielt werden.

Optional folgt auf Schritt (a) die in Figur 1 angegebene Desodorierung (a'). Hierauf wird weiter unten eingegangen.

Die Abtrennung der durch die Aufkonzentrierung entstandenen Ausfällungen (Feststoffe) gemäß Schritt (b) betrifft insbesondere Fremdproteine und/oder inaktive Enzyme, besonders in der Nähe des Löslichkeitsprodukts. Dieser Schritt wird im Blockfließbild der Figur 1 als "Separation" bezeichnet. Sie erfolgt ebenfalls nach an sich bekannten Methoden, beispielsweise über einen Separator (siehe unten), wie dies auch in den Beispielen zur vorliegenden Anmeldung offenbart wird.

In dem das interessierende Enzym enthaltenden Überstand sollte nun möglichst wenig (siehe unten) an Schwebstoffen, das heißt an Feststoffen enthalten sein, die wie oben bereits angeführt über eine Tischzentrifuge bestimmbar sind. Denn als Feststoffe ausgefällte Proteine können durch Verdünnen nicht ohne erheblichen Verlust (siehe oben) und nur unter drastischer Verringerung der Konzentration wieder in Lösung gebracht werden. Zudem beeinträchtigen sie wie auch sonstige Feststoffe den nachfolgenden Chromatographie-Schritt, indem sie die Säule blockieren können.

Schritt (c) stellt mit der Entfärbung des weitgehend feststoffreien Überstands von Schritt (b) über einen starkbasischen Anionenaustauscher (Adsorber) den Kern der Erfindung dar. Bei diesem Schritt adsorbieren vor allem die farbigen Begleitstoffe an den Harz, insbesondere die Maillard-Verbindungen, während durch die stark positive Ladung des Austauschers die unter entsprechend zu wählenden Bedingungen ebenfalls positiv geladenen Proteine nicht auf dem Harz gebunden, sondern mit dem Eluat in einer weitgehend klaren Lösung erhalten werden. Schritt (c) stellt somit eine selektive Abtrennung der Farbstoffe aus der konzentrierten Enzymlösung dar.

Der Vorteil dieses Verfahrens gegenüber den im Stand der Technik beschriebenen Verfahren besteht darin, daß die interessierenden Wertstoffe, das heißt die Enzymproteine in Lösung bleiben, das heißt nicht denaturiert und renaturiert werden müssen und somit nicht in ihrer räumlichen Struktur verändert werden. Sie verbleiben damit auch in der weiter zu prozessierenden Phase und werden nicht aus dem System ausgetragen. Dadurch konnten die oben erwähnten hohen Ausbeuten erreicht werden.

Die an das Harz gebundenen, überwiegend farbigen Stoffe werden, wie dies in Figur 1 durch entsprechende dicke Pfeile angedeutet ist, anschließend, das heißt nach Austritt der Wertstoff-enthaltenden Phase (des Gutprodukts) und gegebenenfalls einem Nachlauf in einem separaten Schritt eluiert. Dies geschieht beispielsweise mit Lösungen mit hoher Ionenstärke, etwa konzentrierte NaCl-Lösungen. Über entsprechende Gegenionen, beispielsweise NaOH, ist das Anionenaustauscher-Material regenerierbar. Je nach Chromatographie-Material können andere einfache Salze besser geeignet sein. Daß dieses Material mit derartigen - zudem kostengünstigen - Verbindungen behandelt werden kann, führt neben dem Reinigungseffekt zu einer Sterilisation. Das System ist somit "CIP"-fähig (für "cleaning in place").

Das Flüssigenzym ist im Anschluß an den Verfahrenschritt (c) weitgehend frei von störenden Schlieren, Ausfällungen und Farbstoffen. Es bleibt auch bei längerer Lagerung bei verschiedenen Temperatur hell, klar und blank und weist gleichzeitig ein hohes Maß an Stabilität auf. Beispiele für erzielbare Farbwerte nach der international gebräuchlichen CIE-Farbskala (definiert in DIN 5033-3 und DIN 6174) gehen aus den in den Beispielen der vorliegenden Anmeldung beschriebenen Versuchen hervor.

Optional schließt sich an den Verfahrensschritt (c) der weiter unten ausführlicher beschriebene Schritt (d) an, nach welchem das hochkonzentrierte Chromatographie-Produkt mit Lösungsmittel gemischt wird. Dies illustriert auch Figur 1 ("Mischung").

Diese nach Schritt (c), beziehungsweise (d) erhaltene, veredelte konzentrierte Enzymlösung ist insbesondere hinsichtlich der farbigen Verunreinigungen deutlich abgreichert, enthält jedoch noch praktisch farblose Begleitstoffe, die aufgrund ihrer zum Teil stabilisierenden Wirkung hochwillkommen sind und aus der konzentrierten Enzymlösung nicht abgetrennt zu werden brauchen/sollen. Zusätzliche Zwischenschritte können je nach Trennproblem vorausgeschickt, eingefügt, angeschlossen oder zusammen mit den genannten Schritten durchgeführt werden. Beispiele für drei solcher optionalen Zwischenschritte werden unten ausgeführt.

Eine weitere Möglichkeit besteht beispielsweise darin, durch einen oder mehrere zusätzliche Chromatographieschritte, insbesondere über andere Trägermaterialien, die im Stand der Technik hinlänglich beschrieben sind (siehe oben) weitere Begleitstoffe aus der konzentrierten Enzymlösung gezielt abzutrennen. Die kann zu jedem im Einzelfall sinnvoll erscheinenden Verfahrenszeitpunkt, vorteilhafterweise unmittelbar vor oder nach dem unter (c) beschriebenen Chromatographieschritt erfolgen, gegebenenfalls voneinander durch Zwischenschritte wie Filtrationen oder Umsolubilisierungen getrennt.

Eine Lösungsmitteländerung, die an verschiedenen Stellen des erfindungsgemäßen Verfahrens, vorzugsweise vor oder anstelle von Schritt (d) vorgenommen werden kann, geht beispielsweise aus der Anmeldung DE 19953870 A1 hervor. Diese offenbart ein Verfahren zur Herstellung einer wasserarmen, ein organisches Lösungsmittel enthaltenen Enzymzubereitung, bei dem eine wäßrige Enzymzubereitung mit einem organischen Lösungsmittel mit einem Siedepunkt von mehr als 100°C vermischt und das Wasser anschließend abdestilliert wird.

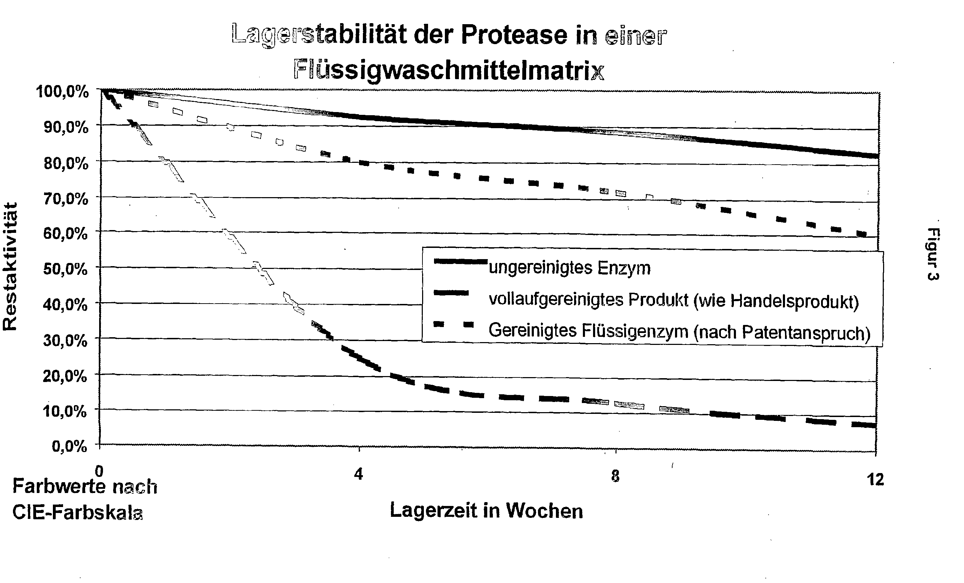

Das nach erfindungsgemäßen Verfahren erhaltene Flüssigenzym kann auf an sich bekannte Weise eingesetzt oder weiterverarbeitet werden. Besondere Bedeutung kommt ihm als Rohstoff zur Einarbeitung in Wasch- oder Reinigungsmittel, insbesondere in flüssiger Form zu. Die erfindungsgemäß geforderte Balance zwischen der klaren Farbe eines solchen Produkts und der mehr als zufriedenstellenden Stabilität veranschaulicht Beispiel 3, dessen Ergebnis auch in Figur 3 dargestellt ist. Man erkennt dort, daß das auf diese Weise veredelte Produkt nur geringfügig instabiler als das ungereinigte Enzym, demgegenüber jedoch nahezu farblos ist und daß es auf der anderen Seite immer noch sehr viel stabiler als ein Handelsprodukt ist, welches auf herkömmliche Weise, nämlich über Fällung entfärbt worden ist.

Im folgenden werden bevorzugte Ausführungsformen und weitere Erfindungsgegenstände ausgeführt.

Wie oben erwähnt, werden in den Verfahrensschritt (a) nach an sich bekannten, im Stand der Technik beschriebenen Verfahren von der Biomasse befreite, angereicherte wäßrige Enzymkonzentrate eingebracht. In der Regel sind dafür mehrere Teilschritte erforderlich. Bevorzugte erfindungsgemäße Verfahren sind dadurch gekennzeichnet, daß als letzter, dem Verfahrensschritt (a) unmittelbar vorangehender Schritt eine Ultrafiltration durchgeführt wird, so daß in den erfindungsgemäßen Schritt (a) ein Ultrafiltrationskonzentrat eingebracht wird. Solch ein Verfahren wird beispielsweise in WO 01/37628 A2 beschrieben. Man erhält dadurch vergleichsweise reine, bereits auf einen mittleren Konzentrationswert angereicherte Enzymlösungen mit einem niedrigen Feststoffgehalt (vergleiche Beispiel 1).

Wie erwähnt werden für die Einkonzentrierung gemäß Schritt (a) an sich bekannte Methoden wie beispielsweise solche mithilfe eines Rotavapors oder eines Dünnschichtverdampfer eingesetzt; letztere Ausführungsform ist bevorzugt.

In einer weiteren bevorzugten Ausführungsform wird Schritt (a) über die jeweils einzustellenden Parameter, insbesondere die Dauer des Konzentrierungsprozesses oder gegebenenfalls Verdünnung so geführt, daß ein Enzym-Konzentrat erhalten wird, welches nicht mehr als 4 bis 20 Gew.-%, vorzugsweise nicht mehr als 4,5 bis 15 Gew.-%, besonders bevorzugt nicht mehr als 5 bis 10 Gew.-% Trockensubstanz enthält.

Bei der Trockensubstanz handelt es sich im Gegensatz zu den oben beschriebenen, unerwünschten Feststoffen um den gesamten Gehalt der konzentrierten Enzymlösung an festen Stoffen, wie sie beispielsweise durch vollständiges Eindampfen der Lösung zu erhalten wären. Diese Werte sind nach an sich bekannten Methoden bestimmbar, beispielsweise über Trocknen eines Aliquots oder über Absorptionsmessung und Vergleich mit einer Eichkurve. Die angegebenen Werte haben sich bei der weiteren Prozessierung als besonders geeignete Werte herausgestellt. Denn zum einen soll die Lösung, um Verluste zu vermeiden, möglichst hochkonzentriert sein, andererseits würde eine zu hohe Viskosität zu Schwierigkeiten im konstanten Durchsatz führen.

In einer weiteren bevorzugten Ausführungsform sind erfindungsgemäße Verfahren dadurch gekennzeichnet, daß in Anschluß an Schritt (a) mit Schritte (a') eine Desodorierung der konzentrierten Enzymlösung durchgeführt wird. Derartige, in einen kontinuierlichen Prozeß integrierbare Desodorierungsmethoden sind aus dem Stand der Technik bekannt. Sie sind dann besonders bevorzugt, wenn es sich bei den für die Herstellung des Enzymproteins eingesetzten Mikroorganismen um solche handelt, die unangenehm riechende Begleitstoffe bilden oder Proteine ausscheiden, die andere Bestandteile sehr rasch abbauen.

Auch der auf (a), beziehungsweise die Desodorierung (a') folgende Verfahrenschritt (b), die Abtrennung von Feststoffen, beruht auf an sich bekannten Methoden, beispielsweise Filtration. Hierunter sind jedoch mechanische, vorzugsweise auf der Schwer- oder Zentrifugalkrafttrennung beruhende Abtrennverfahren bevorzugt.

Dazu gehören insbesondere Separatoren, vorzugsweise kontinuierlich arbeitende Separatoren, die in eine kontinuierliche Prozeßführung eingegliedert werden können. Hirunter sind solche mit einem diskontinuierlichem Sedimentaustrag besonders bevorzugt. Solche offenbaren auch die Beispiele zur vorliegenden Anmeldung.

Der Sinn von Schritt (b) besteht darin, den Gehalt des Enzymkonzentrats an Schweb- oder Feststsoffen auf einen möglichst niedrigen Wert zu reduzieren. Bevorzugte Verfahren sind dadurch gekennzeichnet, daß in der konzentrierten Enzymlösung durch Schritt (b) nicht mehr als 1 Vol.-%, bevorzugt nicht mehr als 0,7 Vol-%, besonders bevorzugt nicht mehr als 0,5 Vol.-% an Feststoff erhalten werden. Dies ist über die an sich bekannte Steuerung der betreffenden Geräte regulierbar; insbesondere die erwähnten Separatoren liefern entsprechend vorteilhafte Lösungen.

Den Kern des erfindungsgemäßen Verfahrens stellt mit Schritt (c) die starkbasische Anionenaustausch-Chromatographie dar. Der Erfolg des Verfahrens hängt deshalb entscheidend von der Art des gewählten Chromatographie-Materials und von der Versuchsführung, beispielsweise dem Probenauftrag ab. Wie bereits ausgeführt, sollen hierbei vor allem die farbigen Begleitstoffe an das Material adsorbieren, während die unter entsprechend zu wählenden Bedingungen positiv geladenen Proteine gerade nicht auf dem Harz gebunden werden. Deshalb beruht die Erfindung auf einem starkbasischen Anionenaustauscher. Aufgrund der Tatsache, daß die meisten natürlichen wasserlöslichen, insbesondere sekretierten Proteine eher bei mittleren pH-Werten wasserlöslich sind, ist es besonders vorteilhaft, wenn der starkbasische Anionenaustauscher für Schritt (c) im pH-Bereich von 5 bis 9, vorzugsweise von 6 bis 8 maximale Austauschkapazität aufweist.

Insbesondere alkalische Proteine, wie beispielsweise die von alkaliphilen Mikroorganismen sekretierten Enzyme, insbesondere Proteasen weisen einen im alkalischen Bereich liegenden isoelektrischen Punkt auf, sind deshalb in dem bevorzugten pH-Bereich positiv geladen und binden deshalb nicht an das betreffende Material. Wie erwähnt, muß für jedes Protein ein für dieses Verfahren idealer pH-Wert experimentell ermittelt und in Hinblick auf diesen Schritt (c) eingestellt werden. In den Beispielen 1 und 2 lagen diese Werte für die gewählte alkalische Protease und die gewählte α-Amylase bei ca. 7,5 und 7.

Als aufgrund ihrer chemischen Eigenchaften besonders geeignet haben sich für Schritt (c) starkbasische Anionenaustauscher herausgestellt, die als funktionelle Gruppen quartäre Ammoniumgruppen aufweisen, vorzugsweise solche, die mit mindestens zwei Alkylgruppen, besonders bevorzugt mit mindestens zwei Alkylgruppen mit 1 oder 2 Kohlenstoffatomen, substituiert sind und optional zusätzlich eine 1 oder 2 Kohlenstoffatomen aufweisende Hydroxyalkylgruppe tragen.

Diese Anforderung wird insbesondere durch starkbasische Anionenaustauscher mit den funktionellen Gruppen Trimethyl-ammonium- oder Dimethylethanol-ammonium-erfüllt. Letzterer ist etwas schwächer basisch als der zuerst genannte, so daß insbesondere über diese Variation auf entsprechende Proteine optimiert werden kann.

Entsprechende Chromatographie-Materialien kennzeichnen dementsprechend bevorzugte Ausführungsformen.

Ein weiteres Kennzeichnen für Chromatographie-Materialien ist ihre Austauschkapazität, die in Mol Äquivalent pro Volumeneinheit angegeben wird. Sie besagt, wie dicht das Material mit den funktionellen Gruppen besetzt ist. Als besonders geeignet haben sich solche Verfahren herausgestellt, die dadurch gekennzeichnet sind, daß der starkbasische Anionenaustauscher für Schritt (c) eine Austauschkapazität von 0,7 bis 1,2 meq/ml vorzugsweise von 0,8 bis 1,1 meq/ml, und besonders bevorzugt von 0,9 bis 1,0 meq/ml aufweist.

Ein weiteres Kriterium, das die Trennleistung der Chromatographie-Säule beeinflußt, ist die effektive Porengröße. Sie muß darauf abgestellt sein, daß sich eine hinreichende Retention ergibt, gleichzeitig aber die hindurchgespülten Stoffe, insbesondere die Proteine nicht zu stark festgehalten werden oder gar das Material verblocken. Für beide in den angegebenen Beispielen untersuchten globulären Proteine B. *lentus*-Alkalische Protease und α-Amylase aus *Bacillus sp.* A 7-7 (DSM 12368), deren Molekulargewicht bei ca. 27 kD, beziehungsweise ca. 58 kD liegt, hat sich ein Chromatographiematerial als brauchbar herausgestellt, dessen effektive Porengröße bei 0,45 mm liegt. Für deutlich größere oder kleinere Proteine sollten Chromatographie-Materialien mit entsprechend größeren oder kleineren effektiven Porengrößen gewählt werden.

Bevorzugte Verfahren sind somit dadurch gekennzeichnet, daß der starkbasische Anionenaustauscher effektive Porengrößen von 0,2 bis 0,7 mm, vorzugsweise von 0,3 bis 0,6 mm, besonders bevorzugt von 0,4 bis 0,5 mm aufweist.

Prinzipiell sind als Träger für erfindungsgemäß einzusetzende starkbasische Anionenaustauscher alle im Stand der Technik hierfür beschriebenen Materialien, beispielsweise auch gelförmige Träger geeignet. Demgegenüber sind aufgrund ihrer technischen Eigenschaften solche starkbasischen Anionenaustauscher für Schritt (c) bevorzugt, die auf einem porösen Kunststoffpolymer beruhen. Als besonders vorteilhaft haben sich solche auf der Basis eines Styrene-DVB-Copolymers herausgestellt.

Chromatographiematerialien, die die soeben diskutierten Eigenschaften aufweisen, sind im Stand der Technik ausführlich beschrieben. Solche aus den DIAION^{®}-Serien werden beipielsweise in dem Handbuch "DIAION^{®}. Manual of ion exchange resins and synthetic adsorbent, Band I" von der Firma Mitsubishi Kasei Corp. (Tokyo, Japan), Juni 1995, auf den Seiten 104 bis 108 und in der "Product Line Brochure DIAION^{®}", Stand 1.6.2001, auf den Seiten 4 bis 6 beschrieben, welche von dem Hersteller, beziehungsweise Summit Chemicals Europe GmbH, Düsseldorf, Deutschland erhältlich ist. Als stark basische Anionenaustauscher werden dort die Serien DIAION^{®}SA, DIAION^{®}PA und DIAION^{®}HPA beschrieben. Ein Vertreter davon, nämlich DIAION^{®}PA308L wurde in den Beispielen zur vorliegenden Anmeldung erfolgreich eingesetzt.

Chemisch ähnliche Materialien, die für die Chromatographie gemäß Schritt (c) einsetzbar sind sind entsprechend den oben gemachten Angaben zu bevorzugten Eigenschaften von entsprechenden Fachleuten herstellbar oder auch von anderen kommerziellen Herstellern erhältlich und charakterisieren ebenso bevorzugte Ausführungsformen. Vergleichbare Ergebnisse werden beispielsweise mit den Materialien DOW MSA Marathon^{®} von der Firma Dow Chemicals und Amberlite^{®}900CL von der Firma Rohm & Haas erzielt.

Zur Durchführung der Chromatographie in Schritt (c) werden vorteilhafterweise bestimmte Bedingungen eingehalten. Hierzu gehören insbesondere ein gewisses Bettvolumen, wobei es sich um das Volumenverhältnis der aufgetragenen Substanz zu dem der Säule handelt, und eine gewisse Verweilzeit, die günstigerweise über die Enzymlösung angegeben wird.

Es hat sich als besonders vorteilhaft herausgestellt und kennzeichnet entsprechend bevorzugte Ausführungsformen, Schritt (c) mit einem Bettvolumen von 1 bis 10, vorzugsweise 1,5 bis 7, besonders bevorzugt 2 bis 4 durchzuführen. Hierbei handelt es sich um das gemäß den Beispielen experimentell ermittelte Optimum, um das Filtrat möglichst rein und gleichzeitig möglichst konzentriert zu halten.

Als geeignete und entsprechend bevorzugte Ausführungsformen kennzeichnende durchschnittliche Verweilzeiten in Schritt (c) wurden dabei Werte von 0,01 bis 0,2 g, vorzugsweise 0,025 bis 0,1 g, besonders bevorzugt 0,04 bis 0,06 g und ganz besonders bevorzugt von 0,05 g Enzym pro g Trägermaterial und Minute ermittelt.

Besonders geeignete Verfahren sind dadurch gekennzeichnet, daß sie weitgehend automatisch gesteuert sind. Eine besonders leicht einzubauende solche Steuerungsmöglichkeit beruht darauf, daß die Leitfähigkeit (meßbar als µS/cm) eines prozessierten Guts an kritischen Stellen ermittelt und zur Steuerung eingesetzt wird. Dies ist beispielsweise nach der Chromatographie möglich und kann dazu genutzt werden, um die wertstoffhaltigen Fraktionen (Gutprodukt) von den übrigen abzutrennen. In einer bevorzugten Ausführungsform ist ein erfindungsgemäßes Verfahren deshalb dadurch gekennzeichnet, daß Schritt (c), insbesondere die Trennung zwischen Vorlauf und Gutprodukt und/oder Gutprodukt und Nachlauf über die Leitfähigkeit des Eluats gesteuert wird.

Zur Ausbeutesteigerung ist bereits in der Anmeldung WO 01/37628 A2 vorgeschlagen worden, Filtrat für einen zusätzlichen Waschschritt einzusetzen. Dementsprechend sind auch Verfahren nach der vorliegenden Erfindung vorzugsweise dadurch gekennzeichnet, daß Schritt (c) unter Rückführung wenigstens eines Teils des Vor- und/oder des Nachlaufs der lonenaustausch-Chromatographie erfolgt. Hierdurch wird eine zusätzliche Anreicherung der betreffenden Fraktion mit solchen Enzymmolekülen erreicht, die gegen Ende des Peaks noch nicht aus der Säule ausgewaschen worden sind. Limitiert wird dieser Schritt prinzipiell durch die möglichen Verunreinigungen, die von der Säule möglicherweise ausgewaschen werden. Im Einzelfall ist zwischen erzielbarer Konzentration und Produktqualität, das heißt Reinheit abzuwägen.

Während des bis hierher beschriebenen Prozesses ist aus Gründen der Effizienz mit einer möglichst hohen Enzymkonzentration gearbeitet worden. Demgegenüber werden konzentrierte Enzymlösungen für ihren technischen Zweck oftmals nicht in derart hohen Konzentrationen benötigt. Entsprechend bevorzugte erfindungsgemäße Verfahren sind deshalb dadurch gekennzeichnet, daß im Anschluß an Schritt (c) in einem Schritt (d) durch Verdünnung ein niedrigerer Konzentrationswert eingestellt wird. Hierfür kommen aus dem Stand der Technik bekannte, insbesondere in ein kontinuierliches System integrierbare Mischer zum Einsatz.

Andererseits neigen alle Flüssigenzyme während der Lagerung dazu, zu denaturieren und dadurch ihre Aktivität zu verlieren. Dies trifft insbesondere auf Proteasen zu, die andere Enzymmoleküle hydrolysieren. Ein bevorzugtes erfindungsgemäßes Verfahren ist somit dadurch gekennzeichnet, daß Anschluß an Schritt (c), ein Stabilisator oder ein Stabilisatorgemisch zugesetzt wird. Solche Verbindungen sind an sich aus dem Stand der Technik bekannt. Es handelt sich beispielsweise um solche, die über Regulation der Wasseraktivität biophysikalisch, beispielsweise gegenüber Temperaturschwankungen stabilisierend wirken, wie Polyole, um solche, die Proteasen reversibel inaktivieren oder die Schutz gegen Oxidation darstellen.

Die Zugabe des Stabilisators, beziehungsweise des Stabilisatorgemischs kann gegebenenfalls vor, nach oder gleichzeitig mit einer Verdünnung (d) erfolgen. Besonders vorteilhaft ist es, wenn eben dieser Schritt (d) genutzt wird, um zusammen mit der Verdünnungslösung eine Stabilisatorlösung oder eine Lösung, die beide Effekte ausübt, zuzusetzen.

Vorzugsweise handelt es sich bei dem zugesetzten Stabilisator um flüssige Verbindungen mit Hydroxylgruppen, beispielsweise ein Polyol wie Glycerin oder besonders bevorzugt um 1,2-Propandiol. Ebenso kann es sich bei derartigen flüssigen Verbindungen um Gemische mit Wasser und/oder weiteren stabilisierenden Verbindungen handeln.

Als besonders günstig zur Entfaltung der Stabilisationswirkung hat sich herausgestellt, wenn die Polyol-Stabilisatormischung in einem Mengenbereich von 40 bis 70 Vol.-%, vorzugsweise von 45 bis 65 Vol.-%, besonders bevorzugt von 50 bis 60 Vol.-% des Endvolumens zugesetzt wird.

Optional kann an dieser Stelle, wenn sich durch die Verdünnung eine zu schwach konzentrierte Lösung ergeben würde, zwischen die Schritte (c) und (d) ein Konzentrierungsschritt geschaltet werden, bevor die Lösung durch den Mischer geleitet wird. Prinzipiell sind hierfür alle aus dem Stand der Technik bekannten, vorzugsweise die oben beschriebenen Methoden anwendbar.

Dies ist auch ein weiteres Argument dafür, während des bisherigen Verfahrens mit einer möglich hochkonzentrierten Enzymlösung zu arbeiten, um durch diesen drastischen Verdünnungseffekt ein Flüssigenzym zu erhalten, das für die beabsichtigten technischen Einsatzgebiete noch hoch genug konzentriert ist.

Dieser Verdünnungsschritt kann ebenfalls dazu genutzt werden, um das Verfahrensendprodukt auf einen Trockensubstanzgehalt von 2 bis 15 Gew.-%, vorzugsweise 5 bis 13 Gew.-%, besonders bevorzugt von 8 bis 12 Gew.-% einzustellen. Ebenso kann er dazu dienen, um das Verfahrensendprodukt auf einen Viskositätswert von 1 bis 20 mPas, vorzugsweise 1 bis 15 mPas, besonders bevorzugt von 1 bis 10 mPas bei 25°C einzustellen und/oder um das Verfahrensendprodukt auf einen Sedimentanteil von weniger als 1 Vol.-%, vorzugsweise weniger als 0,75 Vol.-%, besonders bevorzugt weniger als 0,5 Vol.-% einzustellen. Diese Werte korrelieren in der Regel miteinander und haben sich für die weitere Lagerung und/oder Handhabung von Flüssigenzymen als besonders geeignet herausgestellt. Diese Einstellung ist wie oben ausgeführt im Anschluß an die Chromatographie, beziehungsweise vor Zumischen eines Stabilisators zu berücksichtigen. Erfindungsgemäße Verfahren, die diese Vorgaben erfüllen, sind entsprechend bevorzugt.

Erfindungsgemäß wird von Enzymen gesprochen, deren konzentrierte Lösungen nach erfindungsgemäßen Verfahren veredelt werden. Enzyme stellen bevorzugte Ausführungsformen dar, weil sie einerseits von besonderem technischem Interesse sind und anderseits über ihre spezifischen Aktivitäten detektiert werden können, was insbesondere für die Bestimmung des optimalen Arbeitsbereiches nach den Beispielen 1 und 2 und Figur 2 ausgenutzt werden kann. Nichtsdestoweniger ist dieses Verfahren auf alle wasserlöslichen Proteine anwendbar, sofern sich zu diesen entsprechende Lösungsmittelsysteme und Chromatographiematerialien finden und entsprechende Nachweisreaktionen etablieren lassen. Beispielsweise gilt dies für Peptide, etwa Peptidhormone, Oligopeptide mit pharmakologischer Bedeutung oder für Antikörper. Antikörper kämen beispielsweise auch für die Detektion in Frage. All diese Proteine sollen im Sinne der vorliegenden Erfindung als Enzyme verstanden werden.

Im Mittelpunkt des Interesses stehen jedoch technisch einsetzbare Enzyme im herkömmlichen Sinne. Dabei handelt es sich vorzugsweise um eine Hydrolase oder eine Oxidoreduktase, besonders bevorzugt um eine Protease, Amylase, Cellulase, Hemicellulase, Lipase, Cutinase oder um eine Peroxidase. Erfindungsgemäße Verfahren, die insbesondere über die Ermittlung und Etablierung geeigneter Prozeßbedingungen (siehe oben) zur Verarbeitung derartiger Enzymlösungen konzipiert sind, stellen entsprechend bevorzugte Ausführungsformen der vorliegenden Erfindung dar.

Von besonders großem Interesse, insbesondere für die Herstellung von Wasch- und Reinigungsmitteln sind Proteasen, vorzugsweise alkalische Proteasen, weil diese besonders aktiv sind und in alkalische Rezepturen eingearbeitet werden können. Entsprechend bevorzugte Verfahren sind deshalb solche, die durch entsprechende Proteasen gekennzeichnet sind.

Hierunter sind aufgrund der biochemischen Eigenschaften der erwähnten alkalischen Proteasen solche Verfahren bevorzugt, die dadurch gekennzeichnet sind, daß insbesondere Schritt (c) bei einem pH-Wert von 5 bis 9, vorzugsweise 6 bis 8,5, besonders bevorzugt 7 bis 8 durchgeführt wird.

Solch eine Protease ist auch in den Beispielen 1 und 3 untersucht worden. Dort ist gezeigt worden, daß für die Durchführung eines erfindungsgemäßen Prozesses die Einhaltung eines kritischen Aktivitätswerts notwendig ist, um das Ausmaß an auftretenden Feststoffen möglichst gering zu halten. Entsprechend bevorzugte Verfahren zur Veredelung der genannten konzentrierten Proteaselösungen sind deshalb dadurch gekennzeichnet, daß das Produkt von Schritt (a) auf einen Aktivitätswert von 600.000 bis 900.000, vorzugsweise 650.000 bis 850.000, besonders bevorzugt 700.000 bis 800.000 HPE pro g eingestellt wird. Für diese Regulation, das heißt Konzentration oder gegebenenfalls Verdünnung sind die aus dem Stand der Technik bekannten Parameter der oben ausgeführten Teilschritte, geeignet.

Entsprechend dem oben Gesagten weisen die für den weiteren Einsatz vorgesehnen Endprodukte vorteilhafterweise niedrigere Aktivitätswerte auf, wie sie insbesondere durch Verdünnung mit stabilisierenden Lösungen eingestellt werden können. Bezogen auf Proteasen werden stellen solche Verfahren bevorzugte Ausführungsformen dar, nach denen das Endprodukt auf einen Aktivitätswert von 150.000 bis 500.000, vorzugsweise 175.000 bis 300.000, besonders bevorzugt 200.000 bis 260.000 HPE pro g eingestellt wird.

Ein weiterer technisch bedeutender Enzymtyp sind die α-Amylasen. Sie werden beispielsweise in der Lebensmittelindustrie zur Herstellung von Backwaren verwendet oder aufgrund ihrer Stärke-hydrolysierenden Aktivität Wasch- und Reinigungsmitteln zugesetzt. Entsprechend dem für die Proteasen Gesagten sind alkalische α-Amylase besonders beliebt. Bei entsprechend bevorzugten Verfahren handelt es sich demnach um solche, die für die Aufarbeitung von α-Amylasen konzipiert und durch diese gekennzeichnet sind; vorzugsweise für, beziehungsweise durch solche mit einem alkalischen pH-Optimum.

Wie in Beispiel 2 und 3 ausgeführt sind bevorzugte erfindungsgemäße Verfahren zur Prozessierung von α-Amylasen dadurch gekennzeichnet, daß das Produkt von Schritt (a) auf einen Aktivitätswert von 30.000 bis 50.000 TAU pro g, vorzugsweise 35.000 bis 45.000 TAU pro g eingestellt wird.

Da auch α-Amylasen zumeist in entsprechend niedrigeren Konzentrationen eingesetzt werden und zudem ebenfalls stabilisiert werden sollten, sind ebenso bevorzugte erfindungsgemäße Verfahren dadurch gekennzeichnet, daß das Endprodukt auf einen Aktivitätswert von 4.000 bis 14.000, vorzugsweise 6.000 bis 12.000, besonders bevorzugt 8.000 bis 10.000 TAU pro g eingestellt wird.

Bei einem weiterhin besonders wichtigen technischen Enzymtyp handelt es sich um Cellulasen, die beispielsweise in der Waschmittelindustrie und in der Textilherstellung zur Oberflächenbehandlung von Textilien eingesetzt werden. Somit stellen auch Verfahren, die durch eine Cellulase, vorzugsweise mit einem alkalischen pH-Optimum gekennzeichnet sind, entsprechend bevorzugte Ausführungsformen der vorliegenden Erfindung dar.

Alle bislang ausgeführten Verfahrensparameter spiegeln sich in den jeweiligen Verfahrensprodukten wieder, beispielsweise was die Art des Enzyms, was den Reinheitsgrad, die Art der abgetrennten Verbindungen, die erhaltenen Aktivitäts- oder Stabilitätswerte angeht. Dementsprechend bevorzugt sind auch die über diese erfindungsgemäßen Verfahren erhaltenen Produkte. Einen, neben dem Verfahren weiteren Erfindungsgegenstand stellen somit konzentrierte Enzymlösungen dar, die durch ein zuvor beschriebenes Verfahren erhalten worden sind.

Ein weiterer Erfindungsgegenstand sind Mittel, die ein Enzym enthalten, welches als Zwischenprodukt als konzentrierte Enzymlösung nach dem zweiten Erfindungsgegenstand erhalten worden ist. So ist es beispielsweise möglich, die erfindungsgemäß erhaltenen konzentrierten Enzymlösungen nicht in flüssiger Form einzusetzen sondern in eine trockene, hochreine Form zu überführen. Dies ist beispielsweise über Lyophilisierung oder durch Einarbeitung in ein festes Granulat möglich. Verfahren hierzu sind in Stand der Technik ausführlich beschrieben. In dieser Form können sie über lange Zeit gelagert oder in andere feste Mittel eingearbeitet werden, beispielsweise in feste Wasch- und Reinigungsmittel.

Zu diesem Erfindungsgegenstand zählen somit auch alle denkbaren Reinigungsmittelarten, sowohl Konzentrate als auch unverdünnt anzuwendende Mittel, zum Einsatz im kommerziellen Maßstab, in der Waschmaschine oder bei der Hand-Wäsche, beziehungsweise -Reinigung. Dazu gehören beispielsweise Waschmittel für Textilien, Teppiche, oder Naturfasern, für die nach der vorliegenden Erfindung die Bezeichnung Waschmittel verwendet wird. Dazu gehören beispielsweise auch Geschirrspülmittel für Geschirrspülmaschinen oder manuelle Geschirrspülmittel oder Reiniger für harte Oberflächen wie Metall, Glas, Porzellan, Keramik, Kacheln, Stein, lackierte Oberflächen, Kunststoffe, Holz oder Leder; für solche wird nach der vorliegenden Erfindung die Bezeichnung Reinigungsmittel verwendet. Jegliche Wasch-oder Reinigungsmittelart stellt eine Ausführungsform der vorliegenden Erfindung dar, sofern sie um ein Enzym bereichert ist, welches nach dem erfinsungsgemäßen Verfahren veredelt und entsprechend diesem Erfindungsgegenstand weiterverarbeitet worden ist.

Ausführungsformen der vorliegenden Erfindung umfassen alle nach den Stand der Technik etablierten und/oder alle zweckmäßigen Darreichungsformen der erfindungsgemäßen Wasch- oder Reinigungsmittel. Dazu zählen insbesondere feste, pulverförmige, Mittel, gegebenenfalls auch aus mehreren Phasen, komprimiert oder nicht komprimiert; ferner gehören beispielsweise dazu: Extrudate, Granulate, Tabletten oder Pouches, sowohl in Großgebinden als auch portionsweise abgepackt. Auch flüssige, pastöse oder gelförmige Ausführungsformen sind eingeschlossen, sofern für diese das erfindungsgemäß aufgearbeitete Enzym in einer weiterverarbeiteten Form eingebracht wird.

In einer bevorzugten Ausführungsform enthalten die erfindungsgemäßen Wasch- oder Reinigungsmittel aktive Enzyme in einer Menge von 2 µg bis 20 mg, vorzugsweise von 5 µg bis 17,5 mg, besonders bevorzugt von 20 µg bis 15 mg, ganz besonders bevorzugt von 50 µg bis 10 mg pro Gramm des Mittels.

Neben einem erfindungsgemäß präparierten Enzym und möglicherweise weiteren Enzymen enthält ein erfindungsgemäßes Wasch- oder Reinigungsmittel gegebenenfalls weitere Inhaltsstoffe wie beispielsweise Enzymstabilisatoren, Tenside, z. B. nichtionische, anionische und/oder amphotere Tenside, Bleichmittel, Bleichaktivatoren, Bleichkatalysatoren, Builder, Lösungsmittel, Verdicker und gegebenenfalls als weitere übliche Inhaltsstoffe Sequestrierungsmittel, Elektrolyte, optische Aufheller, Vergrauungsinhibitoren, Farbübertragungsinhibitoren, Schauminhibitoren, Farb- und/ oder Duftstoffe, antimikrobielle Wirkstoffe und/oder UV-Absorbenzien, um nur die wichtigsten Stoffklassen auszuführen. Entsprechende Rezepturen sind im Stand der Technik ausführlich beschrieben.

Demgegenüber sind aufgrund der vorteilhaften Eigenschaften der erfindungsgemäß erhaltenen Flüssigenzyme, insbesondere wegen ihres klaren Erscheinungsbilds und weil sie ohne weitere Aufarbeitung eingesetzt werden können, Mittel bevorzugt, die eine entsprechend konzentrierte Enzymlösung enthalten. Hierbei sind besonders solche Mittel von Interesse, die in insgesamt flüssiger, pastöser oder Gelform vorliegen. Sie können leicht dosiert werden, enthalten das Enzym in der gewünschten Aktivität und weisen, zumindest was die Enzymkomponente angeht, ein ansprechendes Erscheinungsbild auf. Dies ist eingangs als wünschenswert dargestellt worden.

Dies gilt in besonderem Maße für Wasch- und Reinigungsmittel, die für den Endverbraucher konzipiert sind. In einer besonders bevorzugten Form handelt es sich bei den Mitteln dieses Erfindungsgegenstands also um Waschmittel oder um Reinigungsmittel. Diese fallen unter die oben angegebene Definition und können die dort angegebenen Stoffe enthalten. Zudem verfügen sie diesem Erfindungsgegenstand entsprechend über eine insgesamt flüssige, gelförmige oder pastöse Form, in welche die erfindungsgemäßen, veredelten Produkte einfach, das heißt nach an sich bekannten Methoden eingearbeitet werden können.

### Beispiele

### Beispiel 1

### Veredelung einer konzentrierten Proteaselösung

### Abtrennung der Biomasse

Nach der fermentativen Herstellung des Wertstoffs Protease, wie sie in WO 91/02792 A1 beschrieben ist, wurde die Biomasse über die bekannten Methoden Separation, Mikrofiltration und Sterilfiltration praktisch vollständig abgetrennt. Darauf folgte wie in der Anmeldung WO 01/37628 A2 beschrieben, eine Einkonzentrierung, das heißt Entfernung des Lösungsmittels mittels einer Ultrafiltration, bis das Proteasekonzentrat eine Aktivität von 300.000 bis 400.000 HPE pro g aufwies, wie sie nach van Raay, Saran und Verbeek, gemäß der Veröffenlichung "Zur Bestimmung der proteolytischen Aktivität in Enzymkonzentraten und enzymhaltigen Wasch-, Spül- und Reinigungsmitteln" in Tenside (1970), Band 7, S. 125 - 132, bestimmbar ist. Ferner wurde mit einer 30 %igen CaCl₂-Lösung ein pH-Wert von 7,5 eingestellt. Die Lösung wies einen Feststoffanteil von weniger als 1 Vol.-% auf, wie er mittels einer Tisch- oder Laborzentrifuge durch Zentrifugation für 10 min bei 7.000 g bestimmbar ist. Zudem zeigte die enthaltene Protease bei einer Ionenstärke von 1 bis 20 mS/cm eine positive Ladung.

### Bestimmung des optimalen Arbeitsbereichs

Proben der nach Abtrennung der Biomasse erhaltenen Lösung wurden während der Ultrafiltration entnommen (Werte bis zu 400.000 HPE), beziehungsweise wie unten beschrieben weiter über einen Separator aufkonzentriert und in Abhängigkeit von der jeweiligen Aktivität wie oben angegeben der Feststoffanteil ermittelt. Die Aktivitätsmessungen erfolgten jeweils bei einem pH-Wert von 7,5, einer Temperatur von 20°C und einer Ionenstärke von 10 mS/cm. Daraus ergab sich die in Tabelle 1 und in Figur 2 dargestellte Abhängigkeit.

**Tabelle 1: Abhängigkeit des Feststoffanteils von der Aufkonzentrierung an aktiver Protease**

| **Aktivität [1000 HPE /g]** | **Feststoffanteil der Lösung [Vol.%]** |
|---|---|
| 0 | 0,2 |
| 200 | 0,5 |
| 400 | 1,2 |
| 600 | 1,5 |
| 800 | 3,0 |
| 1000 | 7,5 |

Wie daraus zu erkennen ist, steigt der Feststoffanteil einer konzentrierten Proteaselösung oberhalb von ca. 900.000 HPE/g überproportional an, so daß als optimaler Arbeitsbereich mit möglichst hoher Aktivität und gleichzeitig möglichst niedrigem Feststoffanteil der Bereich von ca. 700.000 bis 800.000 HPE/g anzusehen ist.

### Schritt (a): Konzentrierung der Enzymlösung bis zum Arbeitsbereich

Die im vorangegangenen Schritt zuletzt durch Ultrafiltration erhaltene Enzymlösung wurde aufgrund dieses Meßergebnisses mittels eines Dünnschichtverdampfers auf den Wert von 800.000 HPE/g einkonzentriert. Dabei wurden folgende Bedingungen eingehalten: Temperatur des Produktes oberhalb von 35°C, Vakuum von ca. 20 mbar, weitgehend konstanter pH-Wert von ca. 7,5 und Geringhalten des Feststoffanteils des Enzymkonzentrates bei weniger als 3 Vol.-%, damit die Verluste im nachfolgenden Schritt möglichst gering bleiben.

### Schritt (b): Separation der entstandenen Ausfüllungen (Feststoffe)

Die Abtrennung, der durch die Aufkonzentrierung entstandenen Feststoffe (Ausfällungen) erfolgte über mechanische Abtrennung, basierend auf dem Prinzip der Schwer- oder Zentrifugalkrafttrennung. Konkret wurde für die Abtrennung der Feststoffe ein Separator mit diskontinuierlichem Sedimentaustrag verwendet; es handelte sich dabei um das Gerät BTPX 205 von der Firma ALFA LAVAL, mit 11.700 m² Klärfläche, betrieben mit einem g-Wert von 12.800 und einem Durchsatz von 200 l/h. Dadurch wurde ein weitgehend feststoffreies Enzymkonzentrat mit weniger als 0,2 Vol.-% Feststoffgehalt erhalten, was wie oben angegeben bestimmt worden ist. Die Aktivität lag weiterhin bei ca. 800.000 HPE/g.

### Schritt (c): Starkbasische Anionenaustausch-Chromatographie

Die chromatographische Entfärbung erfolgte im Festbett mittels des starkbasischen Anionenaustauschers vom Typ DIAION^{®} Pa 308L, erhältlich von der Firma Mitsubishi, Tokyo, Japan, beziehungsweise Mitsubishi Chemical Europe GmbH, Düsseldorf, Deutschland. Dabei wurde die Entfärbungsqualität und damit die Stabilität des Enzyms über das Bettmengenverhältnis (BM; Volumenverhältnis des Enzymkonzentrats zum Harz) und die Verweilzeit gesteuert; und zwar wurde ein Verhältnis von 2 bis 5 BM und eine Dosierung von 0,05 kg Enzymlösung pro kg Harz und min eingestellt. Das Prinzip beruhte darauf, daß das Enzym vom Trägermaterial abgestoßen, und somit im Flüssigkeitsstrom mitgeführt wird, während die Farbstoffe am immobilen Träger binden. Ein Teil des Nachlaufs wurde zur Erhöhung der Ausbeute erneut über die Säule geführt. Anschließend wurden durch Spülen mit NaCl- und NaOH-Lösungen die an die Säule adsorbierten Verbindungen, insbesondere die Farbstoffe ausgewaschen und auf diese Weise das Festbett regeneriert.

### Schritt (d): Mischung, Stabilisierung und Aktivitätseinstellung mit Lösungsmittel

Das Filtrat mit einem Aktivitätswert von ca. 700.000 HPE pro g wurde unmittelbar, das heißt on line in einem statischen Mischer, mit 1,2-Propandiol gemischt, welches gleichzeitig eine stabilisierende Wirkung zeigt. Es wurden ca. 55 Vol.-% Lösungsmittelanteil genommen.

Das über diese vier Schritte veredelte Flüssigenzym wies folgende Eigenschaften auf, wobei die Aktivität wie oben definiert ermittelt wurde und die nach der international gebräuchlichen CIE-Farbskala, definiert in DIN 5033-3 und DIN 6174, bestimmt wurde:

| | |
|---|---|
| Aktivität: | 260.000 HPE / g |
| Farbe: | L- Wert > 96 |
| | b*-Wert < 14 |
| Viskosität: | < 10 mPas |
| pH-Wert: | ca. 7 |

Die erhaltene Flüssigkeit war praktisch klar und zeigte unmittelbar im Anschluß an das Verfahren keine Nachfällungen. Die weitere Stabilität wird in Beispiel 3 beschrieben.

### Beispiel 2

### Veredelung einer konzentrierten Amylaselösung

Die Herstellung einer erfindungsgemäß veredelten Amylaselösung erfolgte bis auf folgende Unterschiede wie Beispiel 1. Es wurde eine Fermentercharge eingesetzt, die als Wertstoff die α-Amylase enthielt, welche in der Anmeldung WO 02/010356 A2 beschrieben ist. Da die α-Amylase einen anderen isoelektrischen Punkt als die Protease aufweist, wurde bereits vor Schritt (a) ein pH-Wert von 6,25 eingestellt und das ganze Verfahren hindurch ein pH-Wert von 6 bis 6,5 aufrechterhalten, um die Ladung der Amylase positiv zu halten.

### Aktivitätsbestimmung

Zur Bestimmung der amylolytischen Aktivität in TAU wird ein modifiziertes p-Nitrophenylmaltoheptaosid verwendet, dessen endständige Glucoseeinheit durch eine Benzylidengruppe blockiert ist; dieses wird durch Amylase zu freiem p-Nitrophenyl-Oligosaccharid gespalten, welches seinerseits mittels der Hilfesenzyme Glucoamylase und alpha-Glucosidase zu Glucose und p-Nitrophenol umgesetzt wird. Damit ist die Menge an freigesetztem p-Nitrophenol der Amylase-Aktivität proportional. Die Messung erfolgt beispielsweise mit dem Quick-Start^{®}-Testkit der Fa. Abbott, Abott Park, Illinois, USA. Die Absorptionszunahme (405 nm) im Testansatz wird bei 37°C über 3 min gegen einen Blank-Wert mittels Photometer detektiert. Die Kalibrierung erfolgt über einen Enzymstandard von bekannter Aktivität (zum Beispiel Maxamyl^{®}/Purastar^{®} 2900 der Firma Genencor, Palo Alto, CA, USA, mit 2.900 TAU/g). Die Auswertung erfolgt mittels Auftragung der Absorptionsdifferenz dE (405 nm) pro min gegen die Enzymkonzentration des Standards.

### Bestimmung des optimalen Arbeitsbereichs

Während der Ultrafiltration und der nachfolgenden Separation wurden wie in Beispiel 1 unterschiedlich konzentrierte Proben genommen und ebenfalls in Abhängigkeit von der jeweiligen Aktivität der Feststoffanteil ermittelt. Die Messungen erfolgten jeweils bei einem pH-Wert von 6,25, einer Temperatur von 20°C und einer Ionenstärke von 10 mS/cm. Daraus ergab sich die in Tabelle 2 und in Figur 2 dargestellte Abhängigkeit.

**Tabelle 2: Abhängigkeit des Feststoffanteils von der Aufkonzentrierung an aktiver α-Amylase**

| **Aktivität [100 TAU /g]** | **Feststoffanteil der Lösung [Vol.-%]** |
|---|---|
| 0 | 0,5 |
| 100 | 1,0 |
| 200 | 1,5 |
| 300 | 2,5 |
| 400 | 3,2 |
| 500 | 5,0 |

Wie daraus zu erkennen ist, besteht bei der Amylase eine der Protease (siehe oben) vergleichbare Abhängigkeit von Feststoffanteil zur Konzentration an aktivem Enzym; diese ist in Figur 2 in 100 TAU pro g angegeben. Demnach steigt der Feststoffanteil einer konzentrierten Amylaselösung oberhalb von ca. 50.000 TAU/g überproportional an, so daß als optimaler Arbeitsbereich mit möglichst hoher Aktivität und gleichzeitig möglichst niedrigem Feststoffanteil der Bereich von ca. 35.000 bis 45.000 TAU/g anzusehen ist. Erfindungsgemäß sollte deshalb unterhalb dieses Bereich gearbeitet werden.

Durch den analog Beispiel 1 durchgeführten Schritt (a) wurde also ein Aktivitätswert von 35.000 bis 45.000 TAU pro g eingestellt und das weitere Verfahren wie in Beispiel 1, das heißt auch am selben Anionenaustausch-Chromatgraphiematerial durchgeführt. In Schritt (d) wurde in analoger Weise durch Mischen mit 1,2-Propandiol ein Konzentrationswert von 9.000 TAU pro g eingestellt.

Das über diese Schritte veredelte Flüssigenzym wies folgende Eigenschaften auf:

| | |
|---|---|
| Aktivität: | 9.000 TAU / g |
| pH-Wert: | ca. 6,25 |

Die Flüssigkeit war praktisch klar und zeigte wie die der Protease in Beispiel 1 unmittelbar im Anschluß an das Verfahren keine Nachfällungen.

### Beispiel 3

### Lagerstabilität der Protease in einer Flüssigwaschmittelmatrix

Zur Ermittlung der Lagerstabilität einer erfindungsgemäß veredelten Protease, im Vergleich zum ungereinigten Enzym und zu einem Handelsprodukt, jeweils in derselben Matrix aus Flüssigwaschmittel wurden folgende drei Proben hergestellt: (1.) eine nicht veredelte Protease, wie sie gemäß Beispiel 1 nach der Ultrafiltration und vor Schritt (a) vorliegt, (2.) das von der Firma Novozymes, Bagsværd, Dänemark, erhältliche voll aufgereinigte Handelsprodukt Savinase^{®}16.0 LEX und (3.) die gemäß Beispiel 1 veredelte Protease. Alle drei Proben wurden in einer Aktivität von 260.000 HPE pro g in einer Lösung von 55 Vol.-% von 1,2-Propandiol in Wasser vorgelegt und in einem Anteil von 0,4 Vol.-% in eine Flüssigwaschmittelmatrix üblicher Zusammensetzung eingearbeitet:

Die in diese Matrix eingebrachten Protease-Proben wiesen auf der CIE-Farbskala (siehe oben) L-Werte von (1.) 78, (2.) 99, beziehungsweise (3.) 97 auf; das heißt, die erfindungsgemäß veredelte Protease war nahezu genauso klar wie das voll aufgereinigte Produkt. Über einen Zeitraum von 12 Wochen wurden regelmäßig Proben entnommen und wie oben angegeben die Restaktivitäten ermittelt. Hierbei ergaben sich die in Tabelle 3 angegebenen Werte, die in Figur 3 graphisch dargestellt sind.

**Tabelle 3: Lagerstabilität der Protease in einer Flüssigwaschmittelmatrix Angegeben ist jeweils die Restaktivität an HPE in %.**

| **Probe** | **0 Wochen** | **4 Wochen** | **8 Wochen** | **12 Wochen** |
|---|---|---|---|---|
| **nicht veredele Protease** | 100 | 92,5 | 88,5 | 82,5 |
| **Savinase^{®} 16.0 LEX** | 100 | 25,0 | 12,8 | 7,3 |
| **gemäß Beispiel 1 veredelte Protease** | 100 | 80,0 | 72,0 | 60,0 |

Man erkennt, daß die erfindungsgemäß veredelte Protease bei einem nahezu gleichen Farbwert mit dem voll aufgereinigten Produkt deutlich stabiler als dieses ist und daß die erfindungsgemäß veredelte Protease gegenüber dem dunklen ungereinigten Enzym in einer Waschmittelmatrix nur schwach an Aktivität verliert.

### Beschreibung der Figuren

- **Figur 1:**: Blockfließschema zur erfindungsgemäßen Veredelung konzentrierter Enzymlösungen
Dargestellt sind folgende Schritte:
- (a): Konzentrierung der Enzymlösung bis zum Arbeitsbereich, wobei überstehende Lösung abgeführt wird;
- (a'): optionale Desodorierung;
- (b): Separation der entstandenen Ausfällungen (Feststoffe);
- (c): Starkbasische Anionenaustausch-Chromatographie, wobei die an die Säule adsorbierten Verbindungen, insbesondere Farbstoffe durch Spülen mit entsprechenden Medien in separaten Schritten ausgewaschen und die Säule regeneriert werden; und
- (d): Stabilisierung und Aktivitätseinstellung durch Zumischen eines Lösungsmittels.
- **Figur 2:**: Abhängigkeit des Feststoffanteils von der Aufkonzentrierung an aktivem Enzym, bestimmt in den Beispielen 1 und 2
Der Feststoffanteil wird in Vol.-% angegeben, wie er mittels einer Laborzentrifuge über Zentrifugation für 10 min bei 7.000 g bestimmbar ist. Die Aktivität der Protease ist in 1.000 HPE/g und die der α-Amylase in 100 TAU/g angegeben. Hervorgehoben sind die gemäß vorliegender Anmeldung als optimal angesehenen Arbeitsbereiche.
- **Figur 3:**: Lagerstabilität der Protease in einer Flüssigwaschmittelmatrix, bestimmt in Beispiel 3
Ermittelt für:
- 1.: nicht veredelte Protease;
- 2.: das Handelsprodukt Savinase^{®}16.0 LEX der Fa. Novozymes; und
- 3.: gemäß Beispiel 1 veredelte Protease.

## Patentansprüche

1. Verfahren zur Veredelung konzentrierter Enzymlösungen enthaltend farbige Maillard-Verbindungen, umfassend folgende Schritte:
(a) Herstellung einer konzentrierten Enzymlösung,
(b) Abtrennung von Feststoffen, insbesondere von Fremdproteinen und/oder inaktiven Enzymen und
(c) Abtrennung der farbigen Maillard-Verbindungen durch Adsorption an einen starkbasischen Anionenaustauscher, wobei der starkbasische Anionenaustauscher quartäre Ammoniumgruppen als funktionelle Gruppen aufweist, eine Austauschkapazität von 0,7 bis 1,2 meq/ml besitzt, eine effektive Porengröße von 0,2 bis 0,7 mm aufweist und auf einem porösen Kunstoffpolymer als Trägermaterial beruht, wobei der starkbasische Anionenaustauscher in einem pH-Bereich von 5 - 9 maximale Austausch-Kapazität aufweist,
**dadurch gekennzeichnet, dass** in der konzentrierten Enzymlösung durch Schritt (b) nicht mehr als 1 Vol.-% an Feststoff erhalten werden und die Enzymproteine das ganze Verfahren über in Lösung bleiben.

2. Verfahren nach Anspruch 1, **dadurch gekennzeichnet, dass** in Schritt (a) ein Ultrafiltrationskonzentrat eingebracht wird.

3. Verfahren nach Anspruche 1 oder 2, **dadurch gekennzeichnet, dass** Schritt (a) mittels eines Dünnschichtverdampfers durchgeführt wird.

4. Verfahren nach einem der Ansprüche 1 bis 3, **dadurch gekennzeichnet, dass** durch Schritt (a) ein Enzym-Konzentrat hergestellt wird, welches nicht mehr als 4 bis 20 Gew.-%, vorzugsweise nicht mehr als 4,5 bis 15 Gew.-%, besonders bevorzugt nicht mehr als 5 bis 10 Gew.-% Trockensubstanz enthält.

5. Verfahren nach einem der Ansprüche 1 bis 4, **dadurch gekennzeichnet, dass** in Anschluss an Schritt (a) mit Schritt (a') eine Desodorierung der konzentrierten Enzymlösung durchgeführt wird.

6. Verfahren nach einem der Ansprüche 1 bis 5, **dadurch gekennzeichnet, dass** Schritt (b) mit einem mechanischen, vorzugsweise auf der Schwer- oder Zentrifugalkrafttrennung beruhenden Abtrennverfahren durchgeführt wird.

7. Verfahren nach Anspruch 6, **dadurch gekennzeichnet, dass** Schritt (b) mit einem Separator, vorzugsweise einem kontinuierlich arbeitenden Separator, besonders bevorzugt einem mit diskontinuierlichem Probenaustrag durchgeführt wird.

8. Verfahren nach einem der Ansprüche 1 bis 7, **dadurch gekennzeichnet, dass** in der konzentrierten Enzymlosung durch Schritt (b) nicht mehr als 1 Vol.-%, bevorzugt nicht mehr als 0,7 Vol-%, besonders bevorzugt nicht mehr als 0,5 Vol.-% an Feststoff erhalten werden.

9. Verfahren nach einem der Ansprüche 1 bis 8, **dadurch gekennzeichnet, dass** der starkbasische Anionenaustauscher für Schritt (c) im pH-Bereich von 5 bis 9, vorzugsweise von 6 bis 8 maximale Austauschkapazität aufweist.

10. Verfahren nach einem der Ansprüche 1 bis 9, **dadurch gekennzeichnet, dass** der starkbasische Anionenaustauscher für Schritt (c) mit mindestens zwei Alkylgruppen, bevorzugt mit mindestens zwei Alkylgruppen mit 1 oder 2 Kohlenstoffatomen, optional zusätzlich mit einer 1 oder 2 Kohlenstoffatomen aufweisende Hydroxyalkylgruppe substituierte quartäre Ammoniumgruppen als funktionelle Gruppen aufweist.

11. Verfahren nach Anspruch 10, **dadurch gekennzeichnet, dass** der starkbasische Anionenaustauscher für Schritt (c) als funktionelle Gruppen Trimethyl-ammonium oder Dimethylethanol-ammonium-Gruppen aufweist.

12. Verfahren nach einem der Ansprüche 1 bis 11, **dadurch gekennzeichnet, dass** der starkbasische Anionenaustauscher für Schritt (c) eine Austauschkapazität von 0,8 bis 1,1 meq/ml, vorzugsweise 0,9 bis 1,0 meq/ml aufweist.

13. Verfahren nach einem der Ansprüche 1 bis 12, **dadurch gekennzeichnet, dass** der starkbasische Anionenaustauscher für Schritt (c) effektive Porengrößen von 0,3 bis 0,6 mm, bevorzugt 0,4 bis 0,5 mm aufweist.

14. Verfahren nach einem der Ansprüche 1 bis 12, **dadurch gekennzeichnet, dass** der starkbasische Anionenaustauscher für Schritt (c) auf einem Styrene-DVB-Copolymer beruht.

15. Verfahren nach einem der Ansprüche 1 bis 14, **dadurch gekennzeichnet, dass** Schritt (c) mit einem Bettvolumen von 1 bis 10, vorzugsweise 1,5 bis 7, besonders bevorzugt 2 bis 4 durchgeführt wird.

16. Verfahren nach einem der Ansprüche 1 bis 15, **dadurch gekennzeichnet, dass** Schritt (c) bei einer durchschnittlichen Verweilzeit von 0,01 bis 0,2 g, vorzugsweise 0,025 bis 0,1 g, besonders bevorzugt 0,04 bis 0,06 g und ganz besonders bevorzugt von 0,05 g Enzym pro g Trägermaterial und Minute erfolgt.

17. Verfahren nach einem der Ansprüche 1 bis 16, **dadurch gekennzeichnet, dass** Schritt (c), insbesondere die Trennung zwischen Vorlauf und Gutprodukt und/oder Gutprodukt und Nachlauf über die Leitfähigkeit des Eluats gesteuert wird.

18. Verfahren nach einem der Ansprüche 1 bis 17, **dadurch gekennzeichnet, dass** Schritt (c) unter Rückführung wenigstens eines Teils des Vor- und/oder des Nachlaufs der Ionenaustausch-Chromatographie erfolgt.

19. Verfahren nach einem der Ansprüche 1 bis 18, **dadurch gekennzeichnet, dass** im Anschluss an Schritt (c) in einem Schritt (d) durch Verdünnung ein niedrigerer Konzentrationswert eingestellt wird.

20. Verfahren nach einem der Ansprüche 1 bis 19, **dadurch gekennzeichnet, dass** im Anschluss an Schritt (c), gegebenenfalls vor, nach oder gleichzeitig zu einer Verdünnung nach Anspruch 19, ein Stabilisator zugesetzt wird, vorzugsweise gleichzeitig mit Schritt (d).

21. Verfahren nach Anspruch 20, **dadurch gekennzeichnet, dass** es sich bei dem Stabilisator um ein Polyol, vorzugsweise 1,2-Propandiol handelt.

22. Verfahren nach Anspruch 21, **dadurch gekennzeichnet, dass** der Stabilisator in einem Mengenbereich von 40 bis 70 Vol.-%, vorzugsweise von 45 bis 65 Vol.-%, besonders bevorzugt von 50 bis 60 Vol.-% des Endvolumens zugesetzt wird.

23. Verfahren nach einem der Ansprüche 19 bis 22, **dadurch gekennzeichnet, dass** das Verfahrensendprodukt auf einen Trockensubstanzgehalt von 2 bis 15 Gew.-%, vorzugsweise 5 bis 13 Gew.-%, besonders bevorzugt von 8 bis 12 Gew.-% eingestellt wird.

24. Verfahren nach einem der Ansprüche 19 bis 23, **dadurch gekennzeichnet, dass** das Verfahrensendprodukt auf einen Viskositätswert von 0,001 bis 0,02 Ns/m² (1 bis 15 mPas), vorzugsweise 0,001 bis 0,015 Ns/m² (1 bis 15 mPas), besonders bevorzugt von 0,001 bis 0,01 Ns/m² (1 bis 10 mPas) bei 25°C eingestellt wird.

25. Verfahren nach einem der Ansprüche 19 bis 24, **dadurch gekennzeichnet, dass** das Verfahrensendprodukt auf einen Sedimentanteil von weniger als 1 Vol.-%, vorzugsweise weniger als 0,75 Vol.-%, besonders bevorzugt weniger als 0,5 Vol.-% eingestellt wird.

26. Verfahren nach einem der Ansprüche 1 bis 25, **dadurch gekennzeichnet, dass** es sich um ein technisch einsetzbares Enzym handelt, vorzugsweise um eine Hydrolase oder eine Oxidoreduktase, besonders bevorzugt um eine Protease, Amylase, Cellulase, Hemicellulase, Lipase, Cutinase oder um eine Peroxidase.

27. Verfahren nach Anspruch 26, **dadurch gekennzeichnet, dass** es sich um eine Protease, vorzugsweise eine alkalische Protease handelt.

28. Verfahren nach Anspruch 27, **dadurch gekennzeichnet, dass** es insbesondere in Schritt (c) bei einem pH-Wert von 5 bis 9, vorzugsweise 6 bis 8,5, besonders bevorzugt 7 bis 8 durchgeführt wird.

29. Verfahren nach Anspruch 27 oder 28, **dadurch gekennzeichnet, dass** das Produkt von Schritt (a) auf einen Aktivitätswert von 600.000 bis 900.000, vorzugsweise 650.000 bis 850.000, besonders bevorzugt 700.000 bis 800.000 HPE pro g eingestellt wird.

30. Verfahren nach einem der Ansprüche 22 bis 24, **dadurch gekennzeichnet, dass** das Endprodukt auf einen Aktivitätswert von 150.000 bis 500.000, vorzugsweise 175.000 bis 300.000, besonders bevorzugt 200.000 bis 260.000 HPE pro g eingestellt wird.

31. Verfahren nach Anspruch 26, **dadurch gekennzeichnet, dass** es sich um eine α-Amylase, vorzugsweise mit einem alkalischen pH-Optimum handelt.

32. Verfahren nach Anspruch 26 oder 31, **dadurch gekennzeichnet, dass** das Produkt von Schritt (a) auf einen Aktivitätswert von 30.000 bis 50.000 TAU pro g, vorzugsweise 35.000 bis 45.000 TAU pro g eingestellt wird.

33. Verfahren nach einem der Anspruche 31 bis 32, **dadurch gekennzeichnet, dass** das Endprodukt auf einen Aktivitätswert von 4.000 bis 14.000, vorzugsweise 6.000 bis 12.000, besonders bevorzugt 8.000 bis 10.000 TAU pro g eingestellt wird.

34. Verfahren nach Anspruch 26, **dadurch gekennzeichnet, dass** es sich um eine Cellulase, vorzugsweise mit einem alkalischen pH-Optimum handelt.

## Claims

1. A method for refining concentrated enzyme solutions comprising colored Maillard compounds, comprising the following steps:
(a) preparing a concentrated enzyme solution,
(b) removal of solids, particularly foreign proteins and/or inactive enzymes and
(c) removal of the colored Maillard compounds by adsorption on a strongly basic anion exchanger, wherein the strongly basic anion exchanger has quaternary ammonium groups as functional groups, has an exchange capacity of 0.7 to 1.2 meq/ml, has an effective pore size of 0.2 to 0.7 mm and is based on a porous synthetic polymer as support material, wherein the strongly basic anion exchanger has maximum exchange capacity in a pH range of 5 - 9,
wherein not more than 1% by volume of solid are obtained in the concentrated enzyme solution by way of step (b) and the enzyme proteins remain in solution throughout the entire process.

2. The method according to claim 1, wherein an ultrafiltration concentrate is introduced in step (a).

3. The method according to claim 1 or 2, wherein step (a) is carried out by means of a thin film evaporator.

4. The method according to any of claims 1 to 3, wherein an enzyme concentrate is prepared by way of step (a), which comprises not more than 4 to 20% by weight, preferably not more than 4.5 to 15% by weight, particularly preferably not more than 5 to 10% by weight of dry substance.

5. The method according to any of claims 1 to 4, wherein a deodorization of the concentrated enzyme solution is carried out subsequent to step (a) in step (a').

6. The method according to any of claims 1 to 5, wherein step (b) is carried out by a mechanical separation process, preferably based on gravitational or centrifugal separation.

7. The method according to claim 6, wherein step (b) is carried out using a separator, preferably a continuously operating separator, particularly preferably one having intermittent sample collection.

8. The method according to any of claims 1 to 7, wherein not more than 1% by volume, preferably not more than 0.7% by volume, particularly preferably not more than 0.5% by volume of solid are obtained in the concentrated enzyme solution by way of step (b).

9. The method according to any of claims 1 to 8, wherein the strongly basic anion exchanger in step (c) has maximum exchange capacity in the pH range of 5 to 9, preferably of 6 to 8.

10. The method according to any of claims 1 to 9, wherein the strongly basic anion exchanger, in step (c), has as functional groups quaternary ammonium groups substituted with at least two alkyl groups, preferably at least two alkyl groups having 1 or 2 carbon atoms, optionally also with a hydroxyalkyl group having 1 or 2 carbon atoms.

11. The method according to claim 10, wherein the strongly basic anion exchanger in step (c) has trimethylammonium or dimethylethanolammonium groups as functional groups.

12. The method according to any of claims 1 to 11, wherein the strongly basic anion exchanger in step (c) has an exchange capacity of 0.8 to 1.1 meq/ml, preferably 0.9 to 1.0 meq/ml.

13. The method according to any of claims 1 to 12, wherein the strongly basic anion exchanger in step (c) has effective pore sizes of 0.3 to 0.6 mm, preferably 0.4 to 0.5 mm.

14. The method according to any of claims 1 to 12, wherein the strongly basic anion exchanger in step (c) is based on a styrene-DVB copolymer.

15. The method according to any of claims 1 to 14, wherein step (c) is carried out using a bed volume of 1 to 10, preferably 1.5 to 7, particularly preferably 2 to 4.

16. The method according to any of claims 1 to 15, wherein step (c) is carried out at a mean residence time of 0.01 to 0.2 g, preferably 0.025 to 0.1 g, particularly preferably 0.04 to 0.06 g and especially preferably 0.05 g of enzyme per g of support material and minute.

17. The method according to any of claims 1 to 16, wherein step (c), in particular the separation of pre-fraction and good product and/or good product and post-fraction, is controlled by means of the conductivity of the eluate.

18. The method according to any of claims 1 to 17, wherein step (c) is performed by recirculating at least a portion of the pre-fraction and/or post-fraction of the ion exchange chromatography.

19. The method according to any of claims 1 to 18, wherein a lower concentration is established subsequent to step (c) by dilution in a step (d).

20. The method according to any of claims 1 to 19, wherein, subsequent to step (c), a stabilizer is added optionally before, after or at the same time as a dilution according to claim 19, preferably at the same time as step (d).

21. The method according to claim 20, wherein the stabilizer is a polyol, preferably propane-1,2-diol.

22. The method according to claim 21, wherein the stabilizer is added in an amount in the range of 40 to 70% by volume, preferably 45 to 65% by volume, particularly preferably 50 to 60% by volume of the final volume.

23. The method according to any of claims 19 to 22, wherein the end product is adjusted to a dry substance content of 2 to 15% by weight, preferably 5 to 13% by weight, particularly preferably 8 to 12% by weight.

24. The method according to any of claims 19 to 23, wherein the end product is adjusted to a viscosity of 0.001 to 0.02 Ns/m² (1 to 15 mPas), preferably 0.001 to 0.015 Ns/m² (1 to 15 mPas), particularly preferably 0.001 to 0.01 Ns/m² (1 to 10 mPas) at 25°C.

25. The method according to any of claims 19 to 24, wherein the end product is adjusted to a sediment fraction of less than 1% by volume, preferably less than 0.75% by volume, particularly preferably less than 0.5% by volume.

26. The method according to any of claims 1 to 25, wherein the enzyme is a technically applicable enzyme, preferably a hydrolase or an oxidoreductase, particularly preferably a protease, amylase, cellulase, hemicellulase, lipase, cutinase or a peroxidase.

27. The method according to claim 26, wherein the enzyme is a protease, preferably an alkaline protease.

28. The method according to claim 27, wherein said method, in particular in step (c), is carried out at a pH of 5 to 9, preferably 6 to 8.5, particularly preferably 7 to 8.

29. The method according to claim 27 or 28, wherein the product from step (a) is adjusted to an activity of 600 000 to 900 000, preferably 650 000 to 850 000, particularly preferably 700 000 to 800 000 HPE per g.

30. The method according to any of claims 22 to 24, wherein the end product is adjusted to an activity of 150 000 to 500 000, preferably 175 000 to 300 000, particularly preferably 200 000 to 260 000 HPE per g.

31. The method according to claim 26, wherein the enzyme is an α-amylase, preferably having an alkaline pH optimum.

32. The method according to claim 26 or 31, wherein the product from step (a) is adjusted to an activity of 30 000 to 50 000 TAU per g, preferably 35 000 to 45 000 TAU per g.

33. The method according to any of claims 31 to 32, wherein the end product is adjusted to an activity of 4000 to 14 000, preferably 6000 to 12 000, particularly preferably 8000 to 10 000 TAU per g.

34. The method according to claim 26, wherein the enzyme is a cellulase, preferably having an alkaline pH optimum.

## Revendications

1. Procédé de raffinage de solutions enzymatiques concentrées contenant des composés de Maillard colorés, comprenant les étapes suivantes :
(a) la fabrication d'une solution enzymatique concentrée,
(b) la séparation de solides, notamment de protéines étrangères et/ou d'enzymes inactives et
(c) la séparation des composés de Maillard colorés par adsorption sur un échangeur d'anions basique fort, l'échangeur d'anions basique fort comprenant des groupes ammonium quaternaires en tant que groupes fonctionnels, présentant une capacité d'échange de 0,7 à 1,2 méq./ml, une taille de pore effective de 0,2 à 0,7 mm et reposant sur un polymère plastique poreux en tant que matériau support, l'échangeur d'anions basique fort présentant une capacité d'échange maximale dans une plage de pH allant de 5 à 9,
**caractérisé en ce que** pas plus de 1 % en volume de solides sont obtenus dans la solution enzymatique concentrée à l'étape (b) et les protéines enzymatiques restent en solution pendant l'ensemble du procédé.

2. Procédé selon la revendication 1, **caractérisé en ce qu'**un concentré d'ultrafiltration est introduit à l'étape (a).

3. Procédé selon la revendication 1 ou 2, **caractérisé en ce que** l'étape (a) est réalisée au moyen d'un évaporateur à couche mince.

4. Procédé selon l'une quelconque des revendications 1 à 3, **caractérisé en ce qu'**un concentré enzymatique est fabriqué à l'étape (a), qui ne contient pas plus de 4 à 20 % en poids, de préférence pas plus de 4,5 à 15 % en poids, de manière particulièrement préférée pas plus de 5 à 10 % en poids, de substance sèche.

5. Procédé selon l'une quelconque des revendications 1 à 4, **caractérisé en ce qu'**une désodorisation de la solution enzymatique concentrée est réalisée après l'étape (a) par une étape (a').

6. Procédé selon l'une quelconque des revendications 1 à 5, **caractérisé en ce que** l'étape (b) est réalisée par un procédé de séparation mécanique, fondé de préférence sur une séparation par force de pesanteur ou centrifuge.

7. Procédé selon la revendication 6, **caractérisé en ce que** l'étape (b) est réalisée avec un séparateur, de préférence un séparateur fonctionnant en continu, de manière particulièrement préférée avec un déchargement discontinu d'échantillons.

8. Procédé selon l'une quelconque des revendications 1 à 7, **caractérisé en ce que** pas plus de 1 % en volume, de préférence pas plus de 0,7 % en volume, de manière particulièrement préférée pas plus de 0,5 % en volume de solides sont obtenus dans la solution enzymatique concentrée à l'étape (b).

9. Procédé selon l'une quelconque des revendications 1 à 8, **caractérisé en ce que** l'échangeur d'anions basique fort pour l'étape (c) présente une capacité d'échange maximale dans la plage de pH allant de 5 à 9, de préférence de 6 à 8.

10. Procédé selon l'une quelconque des revendications 1 à 9, **caractérisé en ce que** l'échangeur d'anions basique fort pour l'étape (c) comprend des groupes ammonium quaternaires substitués avec au moins deux groupes alkyle, de préférence avec au moins deux groupes alkyle de 1 ou 2 atomes de carbone, éventuellement également avec un groupe hydroxyalkyle comprenant 1 ou 2 atomes de carbone, en tant que groupes fonctionnels.

11. Procédé selon la revendication 10, **caractérisé en ce que** l'échangeur d'anions basique fort pour l'étape (c) comprend des groupes triméthyl-ammonium ou diméthyléthanol-ammonium en tant que groupes fonctionnels.

12. Procédé selon l'une quelconque des revendications 1 à 11, **caractérisé en ce que** l'échangeur d'anions basique fort pour l'étape (c) présente une capacité d'échange de 0,8 à 1,1 méq./ml, de préférence de 0,9 à 1,0 méq./ml.

13. Procédé selon l'une quelconque des revendications 1 à 12, **caractérisé en ce que** l'échangeur d'anions basique fort pour l'étape (c) présente des tailles de pores effectives de 0,3 à 0,6 mm, de préférence de 0,4 à 0,5 mm.

14. Procédé selon l'une quelconque des revendications 1 à 12, **caractérisé en ce que** l'échangeur d'anions basique fort pour l'étape (c) repose sur un copolymère styrène-DVB.

15. Procédé selon l'une quelconque des revendications 1 à 14, **caractérisé en ce que** l'étape (c) est réalisée avec un volume de lit de 1 à 10, de préférence de 1,5 à 7, de manière particulièrement préférée de 2 à 4.

16. Procédé selon l'une quelconque des revendications 1 à 15, **caractérisé en ce que** l'étape (c) a lieu pendant un temps de séjour moyen de 0,01 à 0,2 g, de préférence de 0,025 à 0,1 g, de manière particulièrement préférée de 0,04 à 0,06 g et de manière tout particulièrement préférée de 0,05 g d'enzyme par g de matériau support et par minute.

17. Procédé selon l'une quelconque des revendications 1 à 16, **caractérisé en ce que** l'étape (c), notamment la séparation entre le produit de tête et le produit cible et/ou entre le produit cible et le produit de queue, est gouvernée par la conductivité de l'éluat.

18. Procédé selon l'une quelconque des revendications 1 à 17, **caractérisé en ce que** l'étape (c) a lieu avec recyclage d'au moins une partie du produit de tête et/ou du produit de queue de la chromatographie à échange d'ions.

19. Procédé selon l'une quelconque des revendications 1 à 18, **caractérisé en ce qu'**une concentration plus faible est ajustée par dilution lors d'une étape (d) après l'étape (c).

20. Procédé selon l'une quelconque des revendications 1 à 19, **caractérisé en ce qu'**un stabilisateur est ajouté après l'étape (c), éventuellement avant, après ou pendant une dilution selon la revendication 19, de préférence pendant l'étape (d).

21. Procédé selon la revendication 20, **caractérisé en ce que** le stabilisateur est un polyol, de préférence le 1,2-propanediol.

22. Procédé selon la revendication 21, **caractérisé en ce que** le stabilisateur est ajouté en une quantité dans une plage allant de 40 à 70 % en volume, de préférence de 45 à 65 % en volume, de manière particulièrement préférée de 50 à 60 % en volume du volume final.

23. Procédé selon l'une quelconque des revendications 19 à 22, **caractérisé en ce que** le produit final du procédé est ajusté à une teneur en substance sèche de 2 à 15 % en poids, de préférence de 5 à 13 % en poids, de manière particulièrement préférée de 8 à 12 % en poids.

24. Procédé selon l'une quelconque des revendications 19 à 23, **caractérisé en ce que** le produit final du procédé est ajusté à une valeur de viscosité de 0,001 à 0,02 Ns/m² (1 à 15 mPas), de préférence de 0,001 à 0,015 Ns/m² (1 à 15 mPas), de manière particulièrement préférée de 0,001 à 0,01 Ns/m² (1 à 10 mPas) à 25 °C.

25. Procédé selon l'une quelconque des revendications 19 à 24, **caractérisé en ce que** le produit final du procédé est ajusté à une proportion de sédiment de moins de 1 % en volume, de préférence de moins de 0,75 % en volume, de manière particulièrement préférée de moins de 0,5 % en volume.

26. Procédé selon l'une quelconque des revendications 1 à 25, **caractérisé en ce qu'**il s'agit d'une enzyme utilisable industriellement, de préférence d'une hydrolase ou d'une oxydoréductase, de manière particulièrement préférée d'une protéase, d'une amylase, d'une cellulase, d'une hémicellulase, d'une lipase, d'une cutinase ou d'une peroxydase.

27. Procédé selon la revendication 26, **caractérisé en ce qu'**il s'agit d'une protéase, de préférence d'une protéase alcaline.

28. Procédé selon la revendication 27, **caractérisé en ce qu'**il est réalisé, notamment à l'étape (c), à un pH de 5 à 9, de préférence de 6 à 8,5, de manière particulièrement préférée de 7 à 8.

29. Procédé selon la revendication 27 ou 28, **caractérisé en ce que** le produit de l'étape (a) est ajusté à une valeur d'activité de 600 000 à 900 000, de préférence de 650 000 à 850 000, de manière particulièrement préférée de 700 000 à 800 000 HPE par g.

30. Procédé selon l'une quelconque des revendications 22 à 24, **caractérisé en ce que** le produit final est ajusté à une valeur d'activité de 150 000 à 500 000, de préférence de 175 000 à 300 000, de manière particulièrement préférée de 200 000 à 260 000 HPE par g.

31. Procédé selon la revendication 26, **caractérisé en ce qu'**il s'agit d'une α-amylase, de préférence ayant un pH optimum alcalin.

32. Procédé selon la revendication 26 ou 31, **caractérisé en ce que** le produit de l'étape (a) est ajusté à une valeur d'activité de 30 000 à 50 000 TAU par g, de préférence de 35 000 à 45 000 TAU par g.

33. Procédé selon l'une quelconque des revendications 31 à 32, **caractérisé en ce que** le produit final est ajusté à une valeur d'activité de 4 000 à 14 000, de préférence de 6 000 à 12 000, de manière particulièrement préférée de 8 000 à 10 000 TAU par g.

34. Procédé selon la revendication 26, **caractérisé en ce qu'**il s'agit d'une cellulase, de préférence ayant un pH optimum alcalin.
